# EUROPEAN PATENT SPECIFICATION

(11) **EP 0 445 819 B1**
(45) Date of publication and mention of the grant of the patent: **22.08.2001**
(21) Application number: 91103511.1
(22) Date of filing: 07.03.1991
(51) Int. Cl.: G03F 7/022

(54) **Positive type photoresist composition**
Positiv arbeitende Photolackzusammensetzung
Composition photosensible positive

(30) Priority: 08.03.1990 JP 5765890; 28.03.1990 JP 8002890; 28.03.1990 JP 8002990
(43) Date of publication of application: 11.09.1991
(73) Proprietor: FUJI PHOTO FILM CO., LTD., Kanagawa-ken (JP)
(72) Inventor: Kawabe, Yasumasa, c/o Fuji Photo Film Co., Ltd., Haibara-gun, Shizuoka (JP); Uenishi, Kazuya, c/o Fuji Photo Film Co., Ltd., Haibara-gun, Shizuoka (JP); Tan, Shiro, c/o Fuji Photo Film Co., Ltd., Haibara-gun, Shizuoka (JP)
(74) Representative: Barz, Peter, Dr.

(56) References cited:
- EP-A- 0 295 465
- EP-A- 0 346 808
- EP-A- 0 358 871
- PATENT ABSTRACTS OF JAPAN vol. 13, no. 75 (P-831)(3423) 21 February 1989, & JP-A-63 261256 (NIPPON ZEON CO LTD) 27 October 1988
- PATENT ABSTRACTS OF JAPAN vol. 14, no. 72 (P-1004)(4015) 09 February 1990, & JP-A-01 289946 (SUMITOMO CHEM CO LTD) 21 November 1989
- PATENT ABSTRACTS OF JAPAN vol. 11, no. 181 (P-585)(2628) 11 June 1987, & JP-A-62 010646 (KANTO KAGAKU K.K.) 19 January 1987
- PATENT ABSTRACTS OF JAPAN vol. 10, no. 276 (C-373)(2332) 19 September 1986, & JP-A-61 097278 (MITSUBISHI CHEM IND LTD) 15 May 1986

## Description

The present invention relates to a positive type photoresist composition sensitive to radiation. More particularly, the present invention relates to a photoresist composition for fine work which provides high resolution and sensitivity and an excellent pattern sectional shape.

The positive type photoresist of the present invention is coated on a substrate such as a semiconducting wafer, glass, ceramic and metal by a spin coating method or roller coating method to a thickness of 0.5 to 3 µm. The coated material is then heated and dried. A circuit pattern or the like is imagewise formed in the material through an exposure mask by irradiation with ultraviolet rays. The material is then subjected to development to obtain a positive image. Subsequently, the positive image is used as a mask to effect patterned etching on a substrate. Typical applications of positive type photoresist are production of semiconductors such as IC, production of circuit board such as liquid crystal and thermal head, and photofabrication.

As positive type photoresist compositions there are normally used compositions comprising an alkali-soluble resin and a naphthoquinonediazide compound as the light-sensitive material. Examples of such compositions: include novolak type phenol resin/naphthoquinonediazide-substituted compounds as disclosed in U.S. Patents 3,666,473, 4,115,128, and 4,173,470. Most typical examples of such compositions include novolak resin made of cresol-formaldehyde/trihydroxybenzophenone-1,2-naphthoquinonediazidosulfonic ester as disclosed in L.F. Thompson, "Introduction to Microlithography", ACS, No. 219, pp. 112 - 121.

As a binder, novolak resin can be dissolved in an alkaline aqueous solution without swelling. The novolak resin can also exhibit a high resistance particularly to plasma etching when an image thus produced is used as a mask for etching. Thus, novolak resin is particularly useful in this application. As a light-sensitive material, a naphthoquinonediazide compound itself serves as a dissolution inhibitor for reducing the alkali solubility of novolak resin but is peculiar in that it undergoes decomposition upon irradiation with light to produce an alkali-soluble substance which rather enhances the alkali solubility of novolak resin. Because of the great change in properties by the irradiation with light, the naphthoquinonediazide compound is particularly useful as the light-sensitive material for a positive type photoresist.

From such a perspective, many positive type photoresists comprising novolak resin and naphthoquinonediazide light-sensitive material have heretofore been developed and put into practical use. These positive type photoresists have attained sufficient results in forming lines of a width of as small as 1.5 to 2 µm.

However, integrated circuits have further increased the degree of integration. In the production of semiconductor boards such as for SLSI (super large-seale integration, it has been required to form very fine patterns of 1 µm or less wide. In such application high sensitivity photoresists are desired from the viewpoints of particularly high resolution, high precision in reproducing patterns to exactly copying the shape of exposure mask and high productivity.

It is a recent tendency that the etching process is switched from wet etching process to dry etching process to inprove resolution and enhance the degree of integration in integrated circuits. However, since the dry etching proces is subject to a rise in the temperature of the resist, the resist to be used in this process is required to exhibit a high heat resistance to avoid heat deformation.

Examples of approaches for improving the heat resistance of the resist include the use of a resin free of components with a weight average molecular weight of 2,000 or less as disclosed in JP-A-60-97347 (the term "JP-A" as used herein means an "unexamined published Japanese patent application") and the use of a resin wherein the total content of monomers, dimers and trimers is 10% by weight or less as disclosed in JP-A60-189739.

However, the use of the above mentioned resin free of or having a reduced amount of low molecular components is disadvantageous in that it normally causes a reduction in sensitivity, lowering the throughput in the production of devices.

It has been attempted to improve the sensitivity or developability of a resist composition by incorporating specified compounds in the resist composition. For example, JP-A-61-141441 discloses a positive type photoresist composition containing trihydroxybenzophenone. The use of such a positive type photoresist composition containing trihydroxybenzophenone enables improvements in sensitivity and developability but is disadvantageous in that the incorporation of trihydroxybenzophenone causes deterioration in the heat resistance of the composition.

In approaches as disclosed in JP-A-64-44439, JP-A-1-177032, JP-A-1-280748, and JP-A-2-10350, an aromatic polyhydroxy compound other than trihydroxybenzophenone is used to provide a higher sensitivity without deteriorating the heat resistance. However, these approaches leave to be desired in the improvement in developability.

It is therefore an object of the present invention to provide a positive type photoresist composition which can provide a resist pattern having high resolution and developability and an excellent heat resistance in the production of semiconductor devices.

The inventors made extensive studies paying attention to these objects. As a result, the inventors found that the objects of the present invention can be accomplished with a positive type light-sensitive resin composition comprising an alkali-soluble resin and a

In particular, the objects of the present invention can be accomplished with a positive type photoresist composition comprising a quinonediazide compound, an alkali-soluble resin, and at least one of the compounds represented by formulae (I) to (VI): 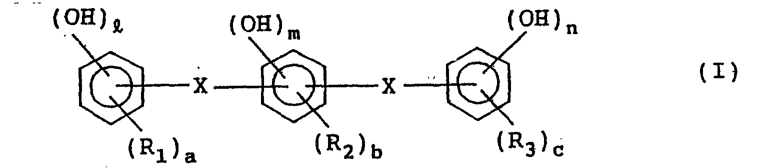 wherein X represents a lower C_{**1-4**} alkylene group; R_{**1**} to R_{**3**} may be the same or different and each represents a hydrogen atom, halogen atom, a C_{**1-4**} alkyl, C_{**1-4**} alkoxy, C_{**2-4**} alkenyl, C_{**2-5**} alkoxycarbonyl, C_{**2-5**} alkyloyloxy, aliphatic C_{**1-5**} acyl, benzoyl or toluoyl group; 1, m and n each represents an integer of from 1 to 3; and a, b and c each represents an integer of from 2 to 4; 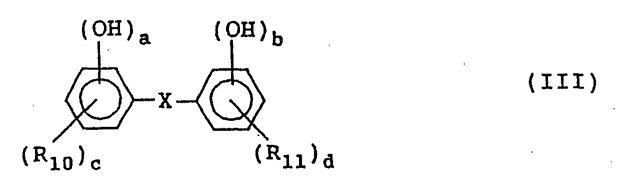 wherein R_{**10**} and R_{**11**} may be the same or different and each represents a hydrogen or halogen atom or a carboxyl, C_{**1-4**} alkyl, phenyl, naphthyl, benzyl, phenethyl, cumyl, benzhydryl, C_{**1-4**} alkoxy, C_{**2-5**} alkoxycarbonyl, C_{**2-5**} alkyloyloxy, aliphatic C_{**1-5**} acyl, benzoyl, toluoyl, phenoxy, naphthoxy, cyano or nitro group each of which may contain substituents; X represents a -CR_{**12**}R_{**13**}- group, 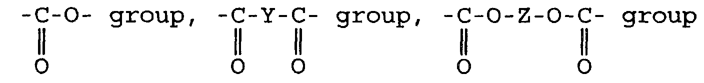 or alkylene group containing 4 or more carbon atoms; Y represents a C_{**1-18**} alkylene group or a benzene residue optionally substituted by C_{**1-4**} alkyl, C_{**1-4**} alkoxy, nitro and halogen; Z represents a C_{**1-4**} alkylene group or an oxyalkylene group derived from ethylene glycol, propylene glycol, polyethylene glycol or polypropylene glycol; R_{**12**} and R_{**13**} each represents (a) an alkyl group containing 5 or more carbon atoms, hydroxyl, phenyl, naphthyl, benzyl, phenethyl, cumyl, benzhydryl, aliphatic C_{**1-5**} acyl, benzoyl or toluoyl group and can form a ring via a carbon bond or ether bond if R_{**10**} and/or R_{**11**} is a hydrogen atom, halogen atom, a C_{**1-4**} alkyl, C_{**1-4**} alkoxy, cyano or nitro group and each group for R_{**12**} and R_{**13**} may contain substituents, or (b) a hydrogen atom, a hydroxyl, C_{**1-10**} alkyl, C_{**1-10**} alkoxy, phenyl, naphthyl, benzyl, phenethyl, cumyl, benzhydryl, aliphatic C_{**1-5**} acyl, benzoyl or toluoyl group and can form a ring via a carbon bond or ether bond if R_{**10**} and R_{**11**} each represents a carboxyl group, phenyl, naphthyl, benzyl, phenethyl, cumyl, benzhydryl, aliphatic C_{**1-5**} acyl, benzoyl, toluoyl, C_{**2-5**} alkoxycarbonyl, C_{**2-5**} alkyloyloxy, phenoxy or naphthoxy group, and each group for R_{**12**} and R_{**13**} may contain substituents; a and b each represents an integer of from 1 to 3, with the proviso that a and b satisfy the relationship 3 ≤ a + b; and c and d each represents an integer of from 1 to 4, with the proviso that a, b, c and d satisfy the relationship a + c = b + d = 5; 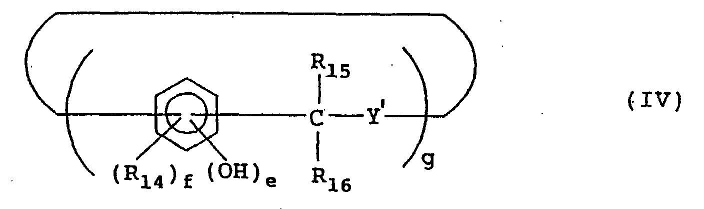 wherein R_{**14**} represents a hydrogen atom, halogen atom, a C_{**1-4**} alkyl, phenyl, naphthyl, benzyl, phenethyl, cumyl, benzhydryl, C_{**1-4**} alkoxy, aliphatic C_{**1-5**} acyl, benzoyl, toluoyl, C_{**2-5**} alkoxycarbonyl, C_{**2-5**} alkyloyloxy, benzoyloxy, toluoyloxy, naphthoyloxy, cyano or nitro group; R_{**15**} and R_{**16**} may be the same or different and each represents a hydrogen atom, a C_{**1-4**} alkyl, phenyl or naphthyl group; Y represents a single bond or -O-CH_{**2**}- group; e and f each represents an integer of from 1 to 3, with the proviso that e and f satisfy the equation e + f = 4; and g represents an integer of from 3 to 8; 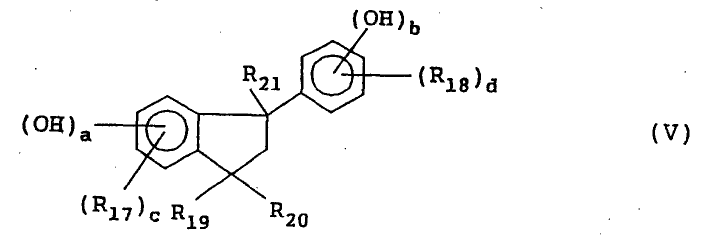 wherein R_{**17**} and R_{**18**} may be the same or different and each represents a hydrogen atom, halogen atom, a C_{**1-4**} alkyl, C_{**1-4**} alkoxy, phenyl, naphthyl, benzyl, phenethyl, cumyl, benzhydryl, amino, C_{**1-4**} monoalkylamino, di- (C_{**1-4**} alkyl)amino, C_{**2-5**} aliphatic acylamino, benzoylamino, toluoylamino, C_{**2-5**} alkylcarbamoyl, phenylcarbamoyl, tolylcarbamoyl, C_{**1-4**} alkylsulfamoyl, carboxyl, cyano, nitro, aliphatic C_{**1-5**} acyl, benzoyl, toluoyl, salicyloyl, naphthoyl, C_{**2-5**} alkyloxycarbonyl, phenoxycarbonyl or aliphatic C_{**2-5**} acyloxy, benzoyloxy, toluoyloxy or naphthoyloxy group each of which may contain substituents; R_{**19**} to R_{**21**} each independently represents a hydrogen atom or a C_{**1-4**} alkyl group which may contain substituents; and a, b, c and d each represents an integer of from 1 to 3, with the proviso that a, b, c and d satisfy the equations a + c = 4 and b + d = 5; 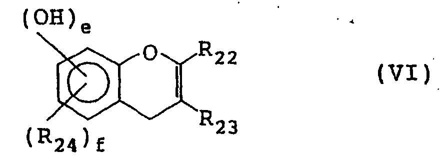 wherein R_{**22**} represents 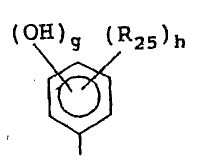 R_{**23**} represents a hydrogen atom, a hydroxyl group, -OR_{**25**} group or 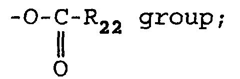 R_{**24**} and R_{**25**} may be the same or different and each represents a hydrogen atom, halogen atom, a C_{**1-4**} alkyl, C_{**1-4**} alkoxy, C_{**2-4**} alkenyl, phenyl, naphthyl, benzyl, phenethyl, cumyl, benzhydryl, aliphatic C_{**1-5**} acyl, benzoyl, toluoyl or C_{**2-5**} alkoxycarbonyl group, each of which may contain substituents; e, f and g may be the same or different and each represents an integer of from 1 to 3; and h represents an integer of from 1 to 4, with the proviso that e, f, g and h satisfy the equations e + f = 4 and g + h = 5; compounds of formula X-A-H, wherein X is a group of the formula: 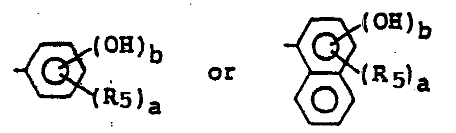 and A is a divalent group of the formula: 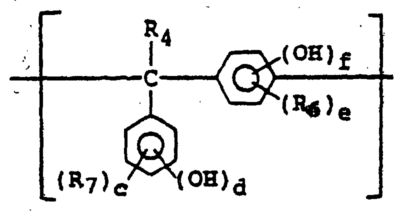 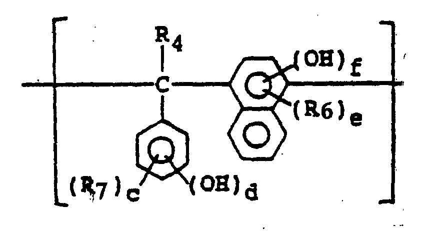 wherein a, b, c, d, e and f are the same or different and a number of 0-3, provided that d+f is not less than 1; R_{**4**} is hydrogen, C_{**1-18**} alkyl or aryl, R_{**5**}**,** R_{**6**} and R_{**7**} are the same or different and represent C_{**1-18**} alkyl, C_{**1-18**} alkoxy, carboxyl or halogen, are excluded from the compounds of formula (III).

The present invention will be further described hereinafter.

The lower alkylene group represented by X in formulae (I) is a C₁₋₄ alkylene group. As R₁ to R₃, preferred examples of halogen include chlorine, bromine and iodine. Preferred examples of C₁₋₄ alkyl are methyl ethyl, propyl, n-butyl, isobutyl, sec-butyl and t-butyl. Preferred examples of C₁₋₄ alkoxy are methoxy ethoxy, hydroxyethoxy, propoxy, hydroxypropoxy, isopropoxy, n-butoxy, isobutoxy, sec-butoxy and t-butoxy. Preferred examples of C₂₋₄ alkenyl are vinyl, propenyl, allyl and butenyl. Preferred examples of C₂₋₅ alkoxycarbonyl are methoxycarbonyl, ethoxycarbonyl, propoxycarbonyl, isopropoxycarbonyl, n-butoxycarbonyl group, isobutoxycarbonyl, sec-butoxycarbonyl and t-butoxycarbonyl. Preferred examples of C₂₋₅ aliphatic alkyloyloxy are acetoxy, propionyloxy, butyryloxy, isobutyryloxy, valeryloxy isovaleryloxy and pivaloyloxy. Preferred examples of C₁₋₅ aliphatic acyl are formyl, acetyl, propionyl, butyryl, valeryl, isovaleryl and pivaloyl.

Specific examples of the compound represented by formula (I) to be used in the present invention will be set forth below, but the present invention should not be construed as being limited thereto.

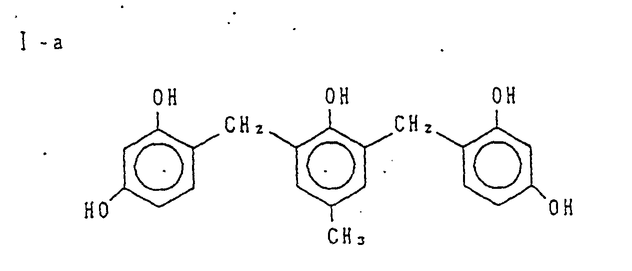

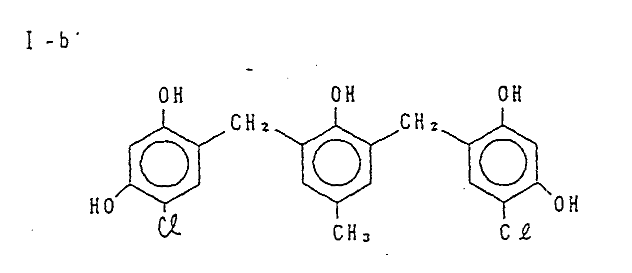

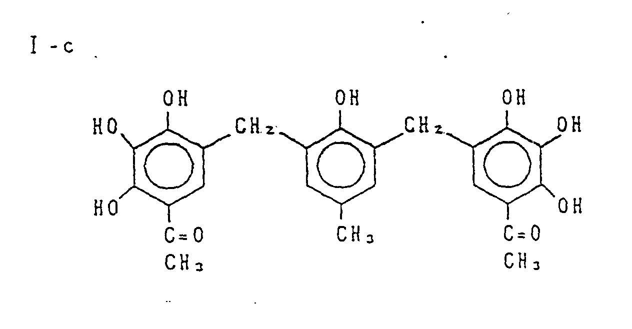

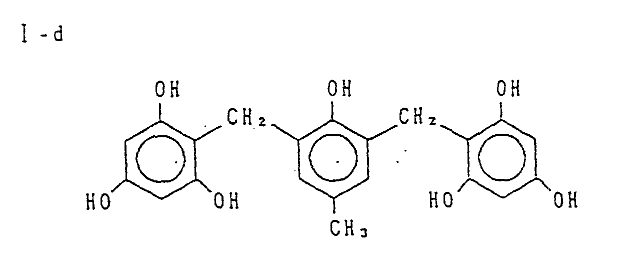

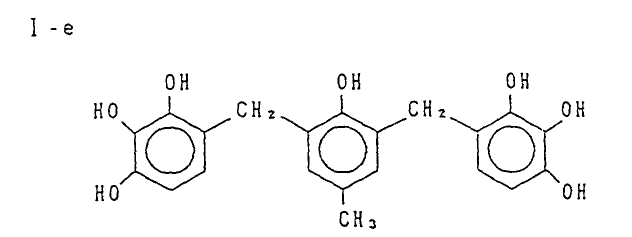

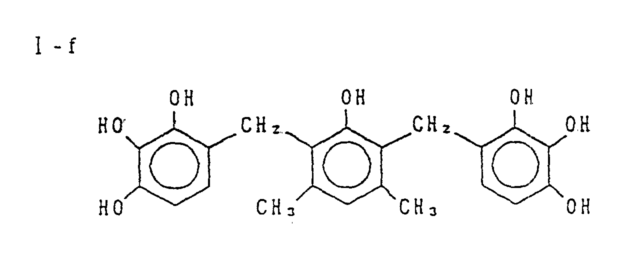

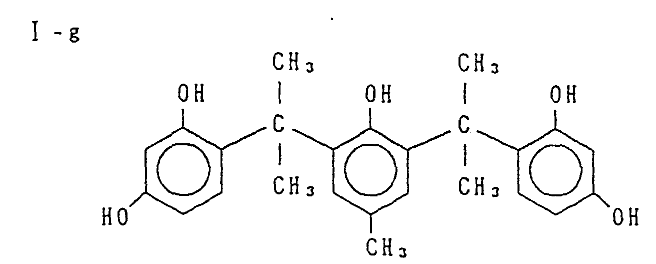

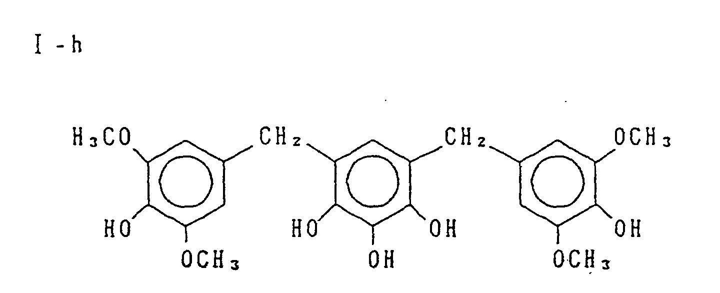

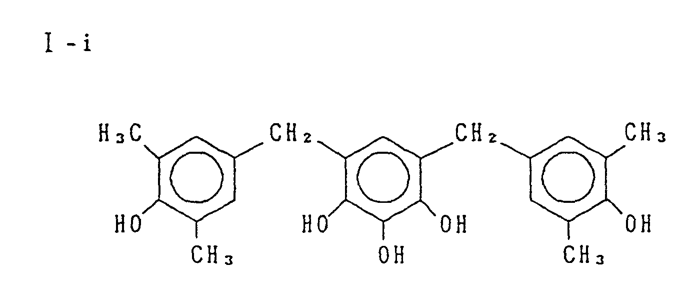

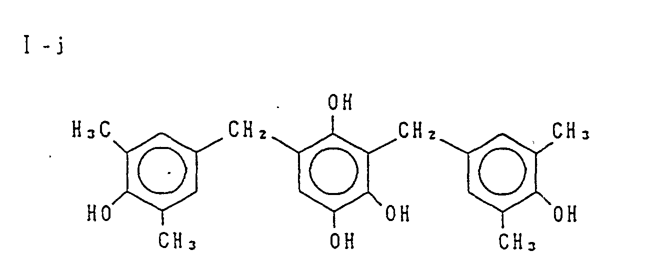

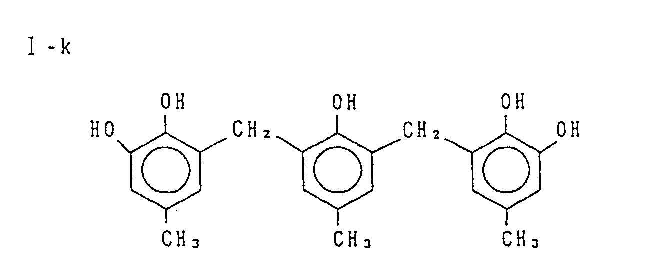

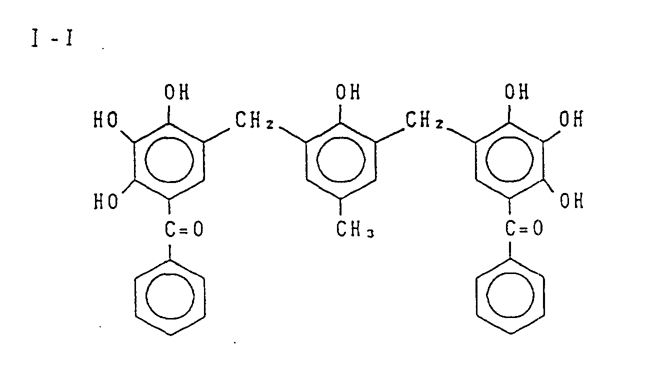

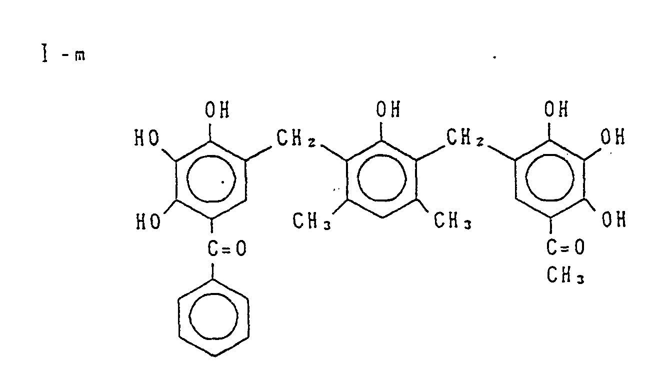

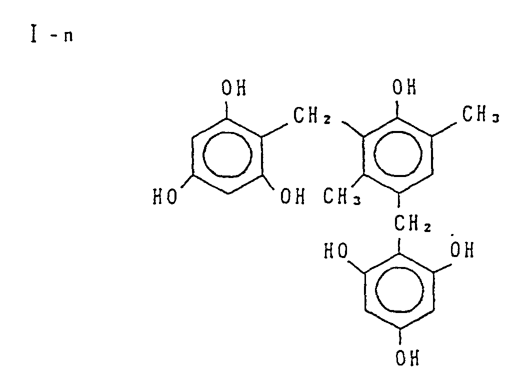

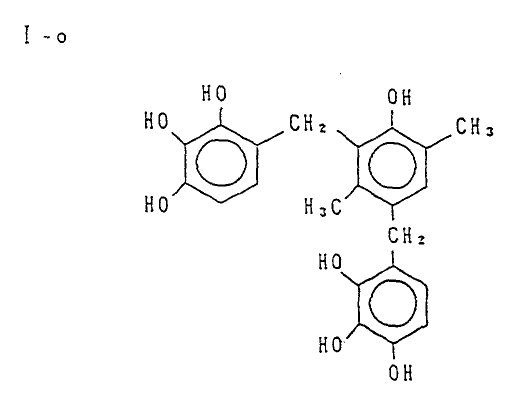

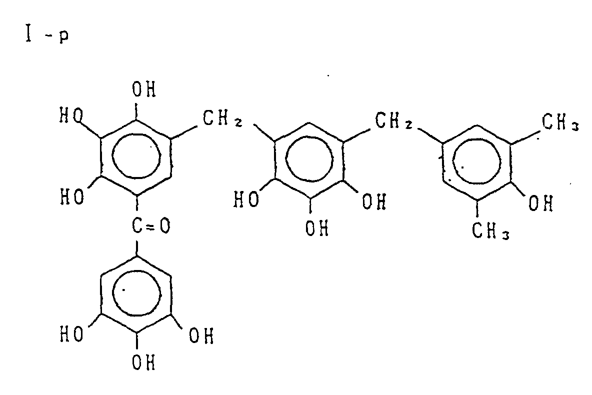

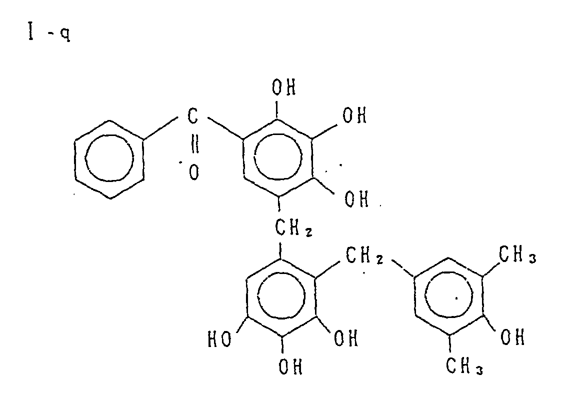

As R₁₀, R₁₁, R₁₅ and R₁₆ in formula (III) or (IV), preferred examples of halogen include chlorine, bromine and iodine. Preferred examples of C₁₋₄ alkyl are methyl, ethyl, propyl, n-butyl isobutyl, sec-butyl and t-butyl. Preferred examples of C₁₋₄ alkoxy are methoxy, ethoxy, hydroxyethoxy, propoxy, hydroxypropoxy, isopropoxy, n-butoxy, isobutoxy, sec-butoxy and t-butoxy. Preferred examples of C₂₋₄ alkenyl are vinyl, propenyl, allyl and butenyl. Preferred examples of C₂₋₅ alkoxycarbonyl are methoxycarbonyl, ethoxycarbonyl, propoxycarbonyl, isopropoxycarbonyl, n-butoxycarbonyl, isobutoxycarbonyl, sec-butoxycarbonyl and t-butoxycarbonyl. Preferred examples of C₂₋₅ aliphatic alkyloyloxy are acetoxy, propionyloxy, butyryloxy, isobutyryloxy, valeryloxy, isovaleryloxy and pivaloyloxy. Preferred examples of C₁₋₅ aliphatic acyl are formyl, acetyl, propionyl, butyryl, valeryl, isovaleryl and pivaloyl.

As R₁₂ and R₁₃ in X of formula (III), preferred examples of alkyl containing 5 or more carbon atoms include pentyl, isopentyl, neopentyl, hexyl, heptyl, octyl, nonyl, and decyl. Preferred examples of acyl include formyl, acetyl, butyryl, benzoyl, cyanamoyl and valeryl.

Specific examples of the compound represented by formula (III) to be used in the present invention include p-bis(2,3,4-trihydroxybenzoyl)benzene, p-bis-(2,4,6-trihydroxybenzoyl)benzene, m-bis(2,3,4-trihydroxybenzoyl)benzene, m-bis(2,4,6-trihydroxybenzoyl)benzene, p-bis(2,5-dihydroxy-3-bromobenzoyl)benzene, p-bis(2,3,4-trihydroxy-5-methylbenzoyl)benzene, p-bis(2,3,4-trihydroxy-5-methoxybenzoyl)benzene, p-bis(2,3,4-trihydroxy-5-nitrobenzoyl)benzene, p-bis(2,3,4-trihydroxy-5-cyanobenzoyl)benzene, benzoyl)benzene, 1,2,4-tris(2,3,4-trihydroxybenzoyl)benzene, 1,2,4,5-tetrakis(2,3,4-trihydroxybenzoyl)benzene, α,α'-bis(2,3,4-trihydroxybenzoyl)-p-xylene, α,α',α'-tris(2,3,4-trihydroxybenzoyl)mesitylene, bis(2,5-dihydroxybenzoyl)methane, bis(2,3,5-trihydroxybenzoyl)methane, ethyleneglycol-di(2-hydroxybenzoate), ethyleneglycol-di(3-hydroxybenzoate), ethyleneglycol-di(4-hydroxybenzoate), ethyleneglycol-di(2,3-dihydroxybenzoate), ethyleneglycol-di(2,6-dihydroxybenzoate), ethyleneglycol-di(3,5-dihydroxybenzoate), ethyleneglycol-di(3,4,5-trihydroxybenzoate), ethyleneglycol-di(2-nitro-3,4,5-trihydroxybenzoate), ethyleneglycol-di(2-cyano-3,4,5-trihydroxybenzoate), ethyleneglycol-di(2,4,6-trihydroxybenzoate), diethyleneglycol-di(2,3-dihydroxybenzoate), 1,3-propanediol-di(3,4,5-trihydroxybenzoate), polytetrahydrofuranglycol-di(3,4,5-trihydroxybenzoate), neopentylglycol-di(3,4,5-trihydroxybenzoate), 1,2-benzenedimethanol-di(3,4,5-trihydroxybenzoate), 1,3-benzenedimethanol-di(3,4,5-trihydroxybenzoate), 2,3,4,2',3',4'-hexahydroxydiphenylcyclohexane-(1,1), 2,3,4,2',3',4'-hexahydroxydiphenyl-4-oxanecyclohexane-(1,1), 2,3,4,2',3',4'-hexahydroxytriphenylmethane, 2,4,6,2',4',6'-hexahydroxydiphenyl-n-butane, 2,4,6,2',4',6'-hexahydroxydiphenyl-n-pentane, 2,4,6,2',4',6'-hexahydroxydiphenylcyclohexane(1,1), 2,4,6,2',4',6'-hexahydroxytriphenylmethane, nordihydroguaiaretinic acid, bis(3-benzoyl-4,5,6-trihydroxyphenyl)methane, bis(3-acetyl-4,5,6-trihydroxyphenyl)methane, bis(3-propionyl-4,5,6-trihydroxyphenyl)methane, bis(3-butyryl-4,5,6-trihydroxyphenyl)methane, bis(3-hexanoyl-4,5,6-trihydroxyphenyl)methane, bis(3-heptanoyl-4,5,6-trihydroxyphenyl)methane, bis(3-heptanoyl-4,5,6-trihydroxyphenyl)methane, bis(3-decanoyl-4,5,6-trihydroxyphenyl)methane, bis(3-octadecanoyl-4,5,6-trihydroxyphenyl)methane, 1,10-bis-(2,4-dihydroxyphenyl)decane-1,10-dione, 1,14-bis-(2,4-dihydroxyphenyl)tetradecane-1,14-dione, 1,8-bis-(2,4-dihydroxyphenyl)octane-1,8-dione, 1,10-bis-(2,3,4-trihydroxyphenyl)decane-1,10-dione, 1,12-bis-(2,4-dihydroxyphenyl)dodecane-1,12-dione, 1,4-bis-(2,4-dihydroxyphe:nyl)butane-1,4-dione, 1,1-(5,5'-diacetyl-2,3,4,2',3',4'-hexahydroxy)diphenylethane, 1,1-(5-acetyl-2,3,4,2',3',4'-hexahydroxy)diphenyl-2-methoxyethane, l,l-(5-acetyl-2,3,4,2',4',6'-hexahydroxy)diphenyl-2-hydroxyethane, 1,1-(2,4,6,2',4'-pentahydroxy-3-propanoyl)diphenylethanol, 4,4',3",4"-tetrahydroxy3,5,3',5'-tetramethyltriphenylmethane, 4,4',2",3",4"-pentahydroxy-3,5,3',5'-tetramethyltriphenylmethane, 2,4,6,2',4',6'-hexahydroxy-5,5'-dipropionyltriphenylmethane, 2,3,4,2',3',4',3",4"-octahydroxy-5,5'-diacetyltriphenylmethane, 2,4,6,2',4',6',2",3",4"-nonahydroxy-5,5'-dipropionyltriphenylmethane, 2,4, 2',4',2",3",4"-heptahydroxytriphenylmethane, 2,3,4-trihydroxybenzoic phenyl, and 3,4,5-trihydroxybenzoic phenyl.

Specific examples of the compound represented by formula (IV) include 10,15-dihydro-2,3,7,8,12,13-hexahydroxy-5H-tribenzo[a,d,g]cyclononene, 10,15-dihydro-1,6,11-trihydroxy-2,7,12-trimethoxy-4,9,14-tripropyl-5H-tribenzo[a,d,g]cyclononene, and 2,8,14,20-tetramethylpentacyclo[19,3,1,1^{3.7},1^{9.13},1^{15.19}]octacosa-1(25),3,5,7(28),9,11,13(27),15,17,19(26),21,23-dodecae n-4,6,10,12,16,18,22,24-octol.

The compound represented by formula (III) or (IV) to be used in the present invention should not be construed as being limited to these compounds.

As R₁₇ and R₁₈ in formula (V), preferred examples of halogen include chlorine, bromine and iodine. Preferred examples of C₁₋₄ alkyl are methyl, ethyl, propyl, n-butyl isobutyl, sec-butyl and t-butyl. Preferred examples of C₁₋₄ alkoxy are methoxy, ethoxy, hydroxyethoxy, propoxy, hydroxypropoxy, isopropoxy, n-butoxy, isobutoxy, sec-butoxy and t-butoxy.

A preferred example of substituted phenyl is hydroxylphenyl. Preferred examples of C₁₋₄ monoalkylamino are monomethylamino, monoethylamino, monopropylamino, monoisopropylamino, mono-n-butylamino, monoisobutylamino, mono-sec-butylamino and mono-t-butylamino. Preferred examples of C₁₋₄ alkyl containing dialkylamino are dimethylamino, diethylamino, dipropylamino, diisopropylamino, di-n-butylamino, diisobutylamino, di-sec-butylamino and di-t-butylamino. Preferred examples of C₂₋₅ aliphatic acylamino are acetylamino, propionylamino, butyrylamino, isobutyrylamino, isovalerylamino, pivaloylamino and valerylamino. Preferred examples of C₂₋₅ alkylcarbamoyl are methylcarbamoyl, ethylcarbamoyl, propylcarbamoyl, isopropylcarbamoyl, n-butylcarbamoyl, isobutylcarbamoyl, sec-butylcarbamoyl and t-butylcarbamoyl. Preferred examples of C₁₋₄ alkylsulfamoyl are methylsulfamoyl, ethylsulfamoyl, propylsulfamoyl, isopropylsulfamoyl, n-butylsulfamoyl, sec-butylsulfamoyl and t-butylsulfamoyl. Preferred examples of C₁₋₅ aliphatic acyl are formyl, acetyl, propionyl, butyryl, isobutyryl, valeryl, isovaleryl and pivaloyl. Preferred examples of C₂₋₅ alkyloxycarbonyl are a methoxycarbonyl, ethoxycarbonyl, propoxycarbonyl, isopropoxycarbonyl, n-butoxycarbonyl, isobutoxycarbonyl, sec-butoxycarbonyl and t-butoxycarbonyl. Preferred examples of C₂₋₅ aliphatic acyloxy are acetoxy, propionyloxy, butyryloxy, isobutyryloxy, valeryloxy, isovaleryloxy and pivaloyloxy.

In R₁₉ to R₂₁, preferred examples of C₁₋₄ alkyl are methyl, ethyl, propyl, isopropyl, butyl, isobutyl, sec-butyl and t-butyl.

As R₂₄ and R₂₅ in formula (VI), preferred examples of halogen include chlorine, bromine and iodine. Preferred examples of C₁₋₄ alkyl are methyl, ethyl, propyl, n-butyl, isobutyl, sec-butyl and t-butyl. Preferred examples of C₁₋₄ alkoxy are methoxy, ethoxy, hydroxyethoxy, propoxy, hydroxypropoxy, isopropoxy, n-butoxy, isobutoxy, sec-butoxy and t-butoxy. Preferred examples of C₂₋₄ alkenyl are vinyl, propenyl, allyl and butenyl. Preferred examples of C₂₋₅ alkoxycarbonyl are methoxycarbonyl, ethoxycarbonyl, propoxycarbonyl, isopropoxycarbonyl, n-butoxycarbonyl, isobutoxycarbonyl, sec-butoxycarbonyl and t-butoxycarbonyl. Preferred examples of C₂₋₅ aliphatic alkyloyloxy are acetoxy, propionyloxy, butyryloxy, isobutyryloxy, valeryloxy, isovaleryloxy and pivaloyloxy. Preferred examples of C₁₋₅ aliphatic acyl are formyl acetyl, propionyl, butyryl, valeryl, isovaleryl and pivaloyl.

Specific examples of the compound represented by formula (V) include 1-(3,4-dihydroxyphenyl)-1,3,3-trimethyl-6-hydroxyindane, 1-(3,4-dihydroxyphenyl)-1,3,3-trimethyl-5,6-dihydroxyindane, 1-(2,3-dihydroxyphenyl)-1,3,3-trimethyl-4,5-dihydroxyindane, -1-(2,3-dihydroxyphenyl)-1,3,3-trimethyl-6,7-dihydroxyindane, 1-(2,4-dihydroxyphenyl)-1,3,3-trimethyl-4,6-dihydroxyindane, 1-(2,4-dihydroxyphenyl)-1,3,3-trimethyl-5,7-dihydroxyindane, 1-(3,5-dihydroxyphenyl)-1,3,3-trimethyl-4,6-dihydroxyindane, 1-(3,5-dihydroxyphenyl)-1,3,3-trimethyl-5,7-dihydroxyindane, 1-(2,3,4-trihydroxyphenyl)-1,3,3-trimethyl-4,5,6-trihydroxyindane, 1-(2,3,4-trihydroxyphenyl)-1,3,3-trimethyl-5,6,7-trihydroxyindane, 1-(2,4,5-tr:ihydroxyphenyl)-1,3,3-trimethyl-4,6,7-trihydroxyindane, 1-(2,4,5-trihydroxyphenyl)-1,3,3-trimethyl-4,5,7-trihydroxyindane, 1-(2,4,6-trihydroxyphenyl)-1,3,3-trimethyl-4,5,6-trihydroxyindane, 1-(3,5-dimethyl-4-hydroxyphenyl)-1,3,3,5,7-pentamethyl-6-hydroxyindane, 1-(2,3,4-trihydroxyphenyl)-1,3,3-triethyl-4,5,6-trihydroxyindane, 1-(2,3,4-trihydroxyphenyl)-1,3,3-triethyl-5,6,7-trihydroxyindane, 1-(2,4-dihydroxyphenyl)-1,3,3-trimethyl-4,6-dihydroxyindane, and 1-(2,4-dihydroxyphenyl)-1,3,3-trimethyl-5,7-dihydroxyindane.

Specific examples of the compound represented by formula (VI) include 2-(3,4-dihydroxyphenyl)-3,5,7-trihydroxybenzopyrane, 2-(3,4,5-trihydroxyphenyl)-3,5,7-trihydroxybenzopyrane, 2-(3,4-dihydroxyphenyl)-3-(3,4,5-trihydroxybenzoyloxy)-5,7-dihydroxybenzopyrane, and 2-(3,4,5-trihydroxyphenyl)-3-(3,4,5-trihydroxybenzoyloxy)-5,7-dihyroxybenzopyrane.

These polyhydroxy compounds can be used singly or in combination.

The proportion of the polyhydroxy compound to the quinonediazide compound is normally in the range of 150 parts by weight or less, preferably 5 to 100 parts by weight per 100 parts by weight of quinonediazide compound. If this value falls below 5 parts by weight, a substantial effect of improving the sensitivity cannot be provided. If this value exceeds 150 parts by weight, the percent film remainining is remarkably lowered.

Examples of alkali-soluble resin to be used in the present invention include novolak resin, acetone-pyrogallol resin, polyhydroxystyrene, and derivatives thereof.

Particularly preferred among these compounds are novolak resin. Such a novolak resin can be obtained by addition condensation of predetermined monomers as main components with aldehydes in the presence of an acidic catalyst.

As such predetermined monomers there can be used, singly or in admixture, phenol; cresols such as m-cresol, p-cresol and o-cresol; xylenols such as 2,5-xylenol, 3,5-xylenol, 3,4-xylenol and 2,3-xylenol; alkylphenols such as m-ethylphenol, p-ethylphenol, o-ethylphenol and p-t-butylphenol; alkoxyphenols such as p-methoxyphenol, m-methoxyphenol, 3,5-dimethoxyphenol, 2-methoxy-4-methylphenol, m-ethoxyphenol, p-ethoxyphenol, m-propoxyphenol, p-propoxyphenol, m-butoxyphenol and p-butoxyphenol, bisalkylphenols such as 2-methyl-4-isopropylphenol, and hydroxyaromatic compounds such as m-chlorophenol, p-chlorophenol, o-chlorophenol, dihydroxybiphenyl, bisphenol A, phenylphenol, resorcinol and naphthol. However, the present invention should not be construed as being limited thereto.

Examples of aldehydes to be used in the addition condensation include formaldehyde, paraformaldehyde, acetaldehyde, propylaldehyde, benzaldehyde, phenylacetaldehyde, α-phenylpropylaldehyde, β-phenylpropyl aldehyde, o-hydroxybenzaldehyde, m-hydroxybenzaldehyde, p-hydroxybenzaldehyde, o-chlorobenzaldehyde, m-chlorobenzaldehyde, p-chlorobenzaldehyde, o-nitrobenzaldehyde, m-nitrobenzaldehyde, p-nitrobenzaldehyde, o-methylbenzaldehyde, m-methylbenzaldehyde, p-methylbenzaldehyde, p-ethylbenzaldehyde, p-n-butylbenzaldehyde, furfural, chloroacetaldehyde and acetal compounds thereof such as chloroacetaldehydediethylacetal. Among these compounds, formaldehyde can be preferably used.

These aldehydes are used singly or in combination.

Examples of acidic catalyst to be used in the addition condensation include hydrochloric acid, sulfuric acid, formic acid, acetic acid, and oxalic acid.

The weight average molecular weight of the novolak resin thus obtained is preferably in the range of 2,000 to 30,000, particularly 6,000 to 20,000. If this value falls below 2,000, the loss of film on unexposed portions after development becomes too great. If this value exceeds 30,000, the development speed is too low.

The weight average molecular weight as specified herein is represented as calculated in terms of polystyrene determined gel permeation chromatography.

The light-sensitive material to be used in the. present invention can comprise a compound obtained by esterification of a polyhydroxy compound as set forth below with 1,2-naphthoquinonediazido-5-(and/or -4-)sulfonylchloride.

Examples of such a polyhydroxy compound include polyhydroxybenzophenones such as 2,3,4-trihydroxybenzophenone, 2,4,4'-trihydroxybenzophenone, 2,4,6-trihydroxybenzophenone, 2,3,4-trihydroxy-2'-methylbenzophenone, 2,3,4,4'-tetrahydroxybenzophenone, 2,2',4,4'-tetrahydroxybenzophenone, 2,4,6,3',4'-pentahydroxybenzophenone, 2,3,4,2',4'-pentahydroxybenzophenone, 2,3,4,2',5'-pentahydroxybenzophenone, 2,4,6,3',4',5'-hexahydroxybenzophenone, and 2,3,4,3',4',5'-hexahydroxybenzophenone; polyhydroxyphenylalkylketones such as 2,3,4-trihydroxyacetophenone, 2,3,4-trihydroxyphenylpentylketone, and 2,3,4-trihydroxyphenylhexylketone; bis((poly)hydroxyphenyl)alkanes such as bis(2,4-dihydroxyphenyl)methane, bis(2,3,4-trihydroxyphenyl)methane, bis(2,4-dihydroxyphenyl)propane-1, bis(2,3,4-trihydroxyphenyl)propane-1, and nordihydroguaialetinic acid; polyhydroxybenzoic esters such as 3,4,5-trihydroxybenzoic propyl, 2,3,4-trihydroxybenzoic phenyl, and 3,4,5-trihydroxybenzoic phenyl; bis(polyhydroxybenzoyl)alkanes or bis(polyhydroxybenzoyl) aryls such as bis(2,3,4-trihydroxybenzoyl)methane, bis(3-acetyl-4,5,6-trihydroxyphenyl)-methane, bis(2,3,4-trihydroxybenzoyl)benzene, and bis(2,4,6-trihydroxybenzoyl) benzene; alkylene-di(polyhydroxybenzoate) such as ethyleneglycol-di(3,5-dihydroxybenzoate) and ethyleneglycol-di(3,4,5-trihydroxybenzoate), polyhydroxybiphenyls such as 2,3,4-biphenyltriol, 3,4,5-biphenyltriol, 3,5,3',5'-biphenyltetrol, 2,4,2',4'-biphenyltetrol, 2,4,6,3',5'-biphenyl-pentol, 2,4,6,2',4',6'-biphenylhexol, and 2,3,4,2',3',4'-biphenylhexol; bis(polyhydroxy)sulfides such as 4,4'-thiobis(1,3-dihydroxy)benzene; bis(polyhydroxyphenyl) ethers such as 2,2',4,4'-tetrahydroxydiphenylether; bis(polyhydroxyphenyl)sulfoxides such as 2,2',4,4'-tetrahydroxydiphenylsulfoxide, bis(polyhydroxyphenyl)sulfones such as 2,2',4,4'-tetrahydroxydiphenylsulfone; polyhydroxytriphenylmethanes such as 4,4',3",4"-tetrabydroxy-3,5,3',5'-tetramethyltriphenylmethane, 4,4',2",3",4"-pentahydroxy-3,5,3',5'-tetramethyltriphenylmethane, 2,3,4,2',3',4'-hexahydroxy-5,5'-diacetyltriphenylmethane, 2,3,4,2',3',4',3",4"-octahydroxy-5,5'-diacetyltriphenyl methane, and 2,4,6,2',4',6'-hexahydroxy-5,5'-dipropionyltriphenylmethane; polyhydroxyspirobiindanes such as 3,3;,3',3'-tetramethyl-1,1'-spirobiindane-5,6,5',6'-tetrol, 3,3,3',3'-tetramethyl-1,1'-spirobiindane-5,6,7,5',6',7'-hexol, 3,3,3',3'-tetramethyl-1,1'-spirobiindane-4,5,6,4',5',6'-hexol, and 3,3,3',3'-tetramethyl-1,1'-spirobi-indane-4,5,6,5',6',7'-hexol, polyhydroxyphthalides such as 3,3-bis(3,4-dihydroxyphenyl)phthalide, 3,3-bis(2,3,4-trihydroxyphenyl)phthalide, and 3',4',5',6'-tetrahydroxyspiro[phthalide-3,9'-xanthene]; polyhydroxybenzopyranes such as 2-(3,4-dihydroxyphenyl)-3,5,7-trihydroxybenzopyrane, 2-(3,4,5-trihydroxyphenyl)-3,5,7-trihydroxybenzopyrane, 2-(3,4-dihydroxyphenyl-3-(3,4,5-trihydroxybenzoyloxy)-5,7-dihydroxybenzopyrane, and 2-(3,4,5-trihydroxyphenyl)-3-(3,4,5-trihydroxybenzoyloxy)-5,7-dihydroxybenzopyrane, and flavono dyes such as morin, quercetin and rutin.

Alternatively, oligomers of phenol resin such as novolak resin can be used.

These light-sensitive materials obtained by esterification of polyhydroxy compound with naphthoquinonediazide can be used singly or in combination.

The proportion of the light-sensitive material to the alkali-soluble resin is normally in the range of 5 to 100 parts by weight, preferably 10 to 50 parts by weight per 100 parts by weight of resin. If this value falls below 5 parts by weight, the percent film remaining is remarkably lowered. If this value exceeds 100 parts by weight, the sensitivity and the solubility in solvent are lowered.

The composition of the present invention can further comprise other polyhydroxy compounds to accelerate solubility in the developer. Preferred examples of such polyhydroxy compounds include phenols, resorcin, phloroglucin, 2,3,4-trihydroxybenzophenone, 2,3,4,4'-tetrahydroxybenzophenone, 2,3,4,3',4',5'-hexahydroxybenzophenone, acetone-pyrogallol condensate resin, phloroglucide, 2,4,2',4'-biphenyltetrol, 4,4'-thiobis(1,3,-dihydroxy)benzene, 2,2',4,4'-tetrahydroxydiphenylether, 2,2',4,4'-tetrahydroxydiphenyl sulfoxide, and 2,2',4,4'-diphenylsulfone.

Such a polyhydroxy compound can be used in an amount of 50 parts by weight or less, preferably 30 parts by weight or less per 100 parts by weight of polyhydroxy compound of the present invention.

Examples of solved in which the light-sensitive material and the alkali-soluble novolak resin can be dissolved include ketones such as methyl ethyl ketone and cyclohexanone; ketoethers such as 4-ethoxy-2-butanone, and 4-methoxy-4-methyl-2-pentanone; alcohol ethers such as ethylene glycol monomethyl ether and ethylene glycol monoethyl ether; ethers such as dioxane and ethylene glycol dimethyl ether; cellosolve esters such as methyl cellosolve acetate and ethyl cellosolve acetate; aliphatic esters such as butyl acetate, methyl lactate and ethyl lactate; halogenated hydrocarbons such as 1,1,2-trichloroethylene; and high polar solvents such as dimethyl acetamide, N-methylpyrrolidone, dimethylformamide and dimethyl sulfoxide. These solvents can be used singly or in admixture with each other.

The positive type photoresist composition of the present invention can comprise surface active agents to further improve coating properties such as striation.

Examples of such surface active agents include nonionic surface active agents such as polyoxyethylene alkyl ethers (e.g., polyoxyethylene lauryl ether, polyoxyethylene stearyl ether, polyoxyethylene cetyl ether, polyoxyethylene oleyl ether); polyoxyethylene alkyl allyl ethers (e.g., polyoxyethylene octyl phenol ether and polyoxyethylene nonyl phenol ether); polyoxyethylene-polyoxypropylene block copolymers; sorbitan aliphatic esters (e.g., sorbitan monolaurate, sorbitan monopalmitate, sorbitan monostearate, sorbitan monooleate, sorbitan trioleate, sorbitan tristearate); and polyoxyethylene sorbitan aliphatic esters (e.g., polyoxyethylene sorbitan monolaurate, polyoxyethylene sorbitan monopalmitate, polyoxyethylene sorbitan monostearate, polyoxyethylene sorbitan trioleate, polyoxyethylene sorbitan tristearate); fluorine-containing surface active agents such as F TOP® EF301, EF303, EF352 (available from Shinakita Kasei K.K.), Megafac® F171, F173 (available from Dainippon Ink And Chemicals, Incorporated), Fluorad® FC430, FC431 (available from Sumito 3M), and Asahi Guard® AG710, Surflon® S-382, SC101, SC102, SC103, SC104, SC105 and SC106 (available from Asahi Glass Company, Limited), Organosiloxane Polymer KP341 (available from The Shin-etsu Chemical Industry Co., Ltd.), and acrylic or methacrylic (co)polymer Polyflow® Nos. 75 and 95 (available from Kyoeisha Yushikagaku Kogyo K.K.). The content of such a surface active agent is normally in the range of 2 parts by weight or less, preferably 1 part by weight or less per 100 parts by weight of alkali-soluble resin and quinonediazide compound in the composition of the present invention.

These surface active agents can be used singly or in combination.

Examples of developer for the positive type photoresist composition of the present invention include aqueous solutions of. alkalies such as inorganic alkali (e.g., sodium hydroxide, potassium hydroxide, sodium carbonate, sodium silicate, sodium metasilicate, aqueous ammonia); primary amines (e.g., ethylamine, n-propylamine); secondary amines (e.g., diethylamine, di-n-butylamine); tertiary amines (e.g., triethylamine, methyldiethylamine); alcoholamines (e.g., dimethylethanolamine, triethanolamine); quaternary ammonium salts (e.g., tetramethylammonium hydroxide, tetraethylammonium hydroxide); and cyclic amines (e.g., pyrrole, piperidine). These aqueous solutions of alkalis may further contain alcohols and surface active agents in proper amounts.

The positive type photoresist composition of the. present invention can comprise a dye, a plasticizer, and a coating aid as necessary. Specific examples of such additives include dyes such as methyl violet, crystal violet and malachite green; plasticizers such as stearic acid, acetal resin, phenoxy resin, and alkyd resin; and adhesion aids such as hexamethyl disilazane, and chloromethyl silane.

The. above mentioned positive type photoresist composition can be coated by a proper coating means such as spinner and coater on a substrate as commonly used in the production of precision integrated circuit elements (e.g., silicon/silicon dioxide-coated substrate), exposed to light through a predetermined mask, and then developed to obtain an excellent resist.

The positive type photoresist of the present invention exhibits excellent resolution, sensitivity, developability and heat resistance and can be thus preferably used as photoresist for fine work.

The present invention will be further described hereinafter, but the present invention should not be construed as being limited thereto. The unit % indicates percent by weight unless otherwise defined.

### EXAMPLES 1 - 6 & COMPARATIVE EXAMPLES 1 - 5

### (1) Synthesis of novolak resin (a)

40 g of m-cresol, 60 g of p-cresol, 54.0 g of formalin (a 37% aqueous solution of formaldehyde) and 0.05 g of oxalic acid were charged into a three-necked flask. The reaction system was then heated with stirring to a temperature of 100°C where it was then allowed to undergo reaction. After the reaction was completed, the reaction system was cooled to room temperature where it was then subjected to reduced pressure of 30 mmHg.

The reaction system was then gradually heated to a temperature of 150°C to remove water and unreacted monomers therefrom. The resulting novolak resin exhibited an average molecular weight of 7,900 as calculated in terms of polystyrene.

### (2) Synthesis of novolak resin (b)

A cresol novolak resin (molecular weight: 9,400 as calculated in terms of polystyrene) was prepared in the same manner as in the foregoing paragraph (1) except that 50% by weight of metacresol, 50% by weight of paracresol and formalin were used. The cresol novolak resin thus obtained was then subjected to separation of low molecular components in accordance with the method as disclosed in Masayoshi Kinoshita and Takayuki Otsu, "Experimental Synthesis of High Molecular Compounds", Kagaku Dojin, page 32, 1973 to obtain a cresol novolak resin with a molecular weight of 10,060 as calculated in terms of polystyrene.

### (3) Synthesis of light-sensitive material (a)

11.5 g of 2,3,4-trihydroxybenzophenone, 30.2 g of 1,2-naphthoquinonediazido-5-sulfonyl chloride and 30 ml of acetone were charged into a three-necked flask, in which they were uniformly dissolved. A mixture of 11.4 g of triethylamine and 50 ml of acetone was gradually added dropwise to the reaction system at a temperature of 25°C for 3 hours. The reaction mixture was poured into 1,500 ml of 1% aqueous hydrochloric acid. The resulting precipitate was filtered off, washed with water, and then dried at a temperature of 40°C to obtain 29.8 g of 1,2-naphthoquinonediazido-5-sulfonic ester of 2,3,4-trihydroxybenzophenone.

### (4) Synthesis of light-sensitive material (b)

12.3 g of 2,3,4,4'-tetrahydroxybenzophenone, 40.3 g of 1,2-naphthoquinonediazido-5-sulfonyl chloride and 300 ml of acetone were charged into a three-necked flask, in which they were uniformly dissolved. A mixture of 15.2 g of triethylamine and 50 ml of acetone was gradually added dropwise to the reaction system at a temperature of 25°C for 3 hours. The reaction mixture was poured into 1,500 ml of 1% aqueous hydrochloric acid. The resulting precipitate was filtered off, washed with water, and then dried at a temperature of 40°C to obtain 39.7 g of 1,2-naphthoquinonediazido-5-sulfonic ester of 2,3,4,4'-tetrahydroxybenzophenone.

### (5) Preparation and evaluation of positive type photoresist composition

Cresol novolak resins (a) and (b) obtained in the foregoing paragraphs (1) and (2) and light-sensitive materials (a) and (b) obtained in the foregoing paragraphs (3) and (4) and additives (a) to (h) indicated in Table 1 were each dissolved in 15 g of ethylcellosolve acetate in amounts indicated in Table 2, respectively. These solutions were then filtered through a 0.2-µm pore microfilter. The resulting photoresist compositions were each coated by a spinner on a silicon wafer, and then dried at a temperature of 90°C in an atmosphere of nitrogen in a convection oven for 30 minutes to obtain 1.2-µm thick resist films. These resist films were each exposed to light through a test chart mask by means of a reduced projection exposure apparatus FPA-1550 available from Canon Inc., developed with a 2.38% aqueous solution of tetramethylammonium hydroxide for 1 minute, washed with water for 30 seconds, and then dried.

The resulting resist patterns on the silicon wafers were then evaulated under a scanning electron microscope. The results are set forth in Table 3.

The sensitivity is defined as reciprocal of the exposure at which a 0.70-µm mask pattern is reproduced. This value is represented relative to that of Comparative Example 1.

The percent film remaining is represented by percent ratio of presence of film before development to that after development.

The resolution is represented by threshold resolution at the exposure at which a 0.70-µm mask pattern is reproduced.

The shape of resist is represented by the angle (θ) formed by the wall surface of the resist and the plane of the silicon wafer in a 0.70-µm thick resist pattern section.

For the evaluation of developability, E indicates an excellent level in which no surface layer peeling and film residue are observed, P indicates a poor level in which much surface layer peeling and film residue are observed, and F indicates a fair level in which slight surface layer peeling and film residue is observed.

The heat resistance is represented by the highest temperature at which a resist pattern on the silicon wafer shows no deformation when the wafer is baked in a convection oven for 30 minutes.

The results which are set forth in Table 3, show that the resists comprising the additives (a) to (c) of the present invention exhibit excellent sensitivity, percent film remaining, resolution, heat resistance, resist shape, and resolution.

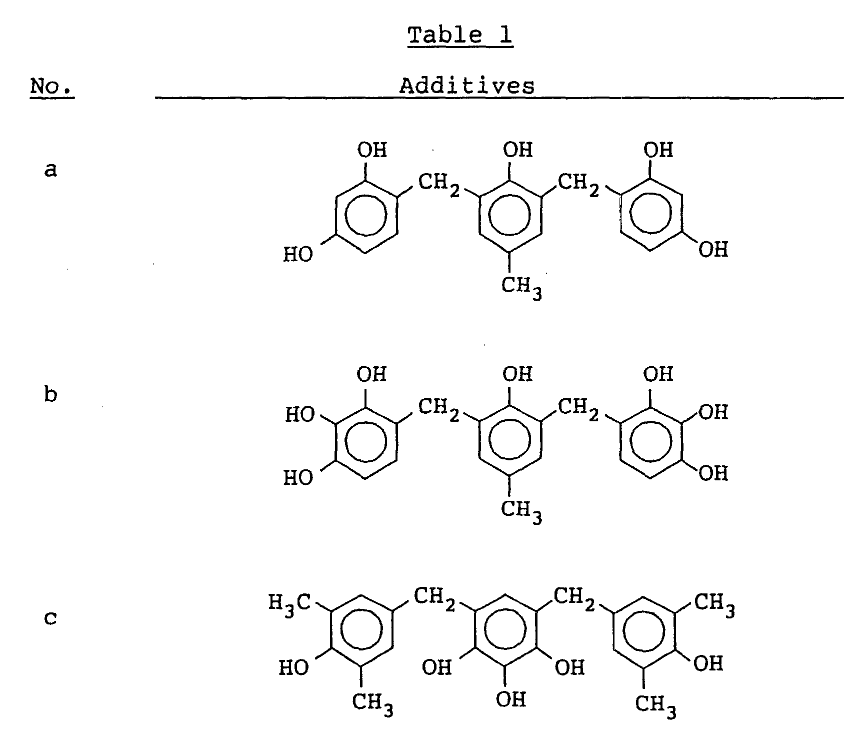

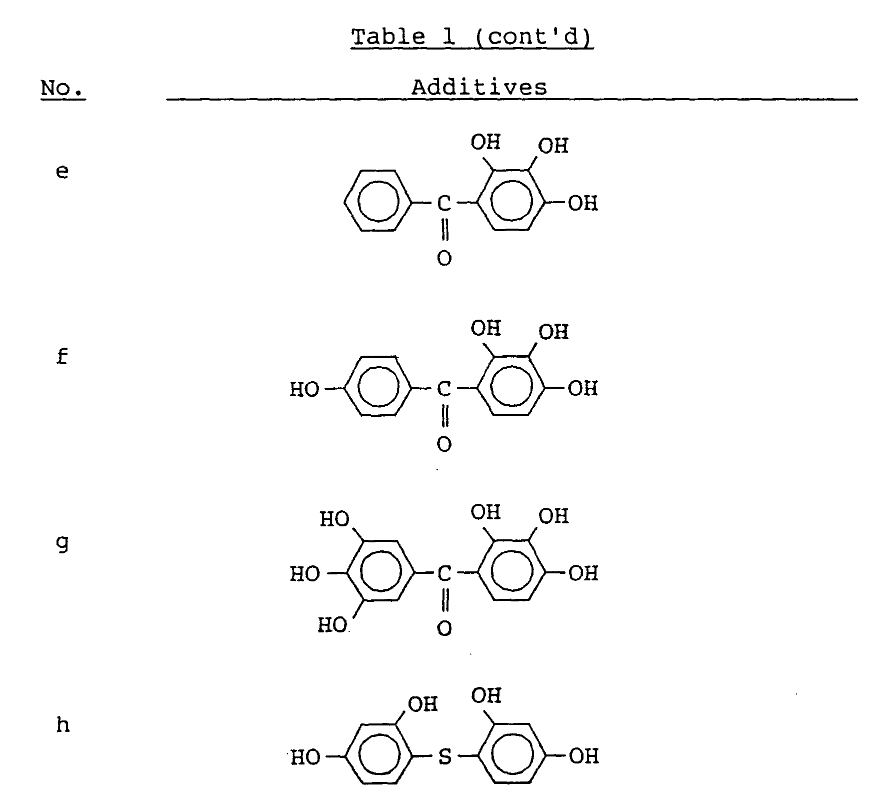

**Table 2:**

| Composition of Resist | | | | | | |
|---|---|---|---|---|---|---|
| | Novolak resin | | Light-sensitive material | | Additive | |
| Example No. | Type | Added amount | Type | Added amount | Type | Added amount |
| | | (g) | | (g) | | (g) |
| Example 1 | (a) | 5.0 | (a) | 1.25 | (a) | 0.38 |
| Example 2 | (a) | 5.0 | (a) | 1.25 | (b) | 0.31 |
| Example 3 | (a) | 5.0 | (b) | 1.25 | (c) | 0.38 |
| Example 4 | (a) | 5.0 | (b) | 1.35 | (a) | 0.41 |
| Example 5 | (b) | 5.0 | (a) | 1.40 | (b) | 0.41 |
| Example 6 | (b) | 5.0 | (b) | 1.40 | (c) | 0.41 |
| Comparative Example 1 | (a) | 5.0 | (a) | 1.25 | None | --- |
| Comparative Example 2 | (a) | 5.0 | (a) | 1.25 | (e) | 0.38 |
| Comparative Example 3 | (a) | 5.0 | (b) | 1.25 | (f) | 0.38 |
| Comparative Example 4 | (b) | 5.0 | (b) | 1.35 | (g) | 0.41 |
| Comparative Example 5 | (b) | 5.0 | (a) | 1.50 | (h) | 0.45 |

**Table 3:**

| Results of Evaluation | | | | | | |
|---|---|---|---|---|---|---|
| Example No. | Relative sensitivity | % Film remaining | Resolution | Heat resistance | Resist shape | Developability |
| | | | (µm) | (°C) | (θ) | |
| Example 1 | 1.1 | 98 | 0.50 | 135 | 86 | E |
| Example 2 | 1.2 | 98 | 0.50 | 135 | 86 | E |
| Example 3 | 1.2 | 99 | 0.50 | 135 | 87 | E |
| Example 4 | 1.2 | 98 | 0.50 | 135 | 86 | E |
| Example 5 | 1.1 | 99 | 0.50 | 140 | 87 | E |
| Example 6 | 1.1 | 99 | 0.50 | 145 | 87 | E |
| Comparative Example 1 | 1.0 | 98 | 0.52 | 135 | 85 | P |
| Comparative Example 2 | 1.1 | 95 | 0.52 | 125 | 82 | E |
| Comparative Example 3 | 1.1 | 97 | 0.52 | 130 | 83 | F |
| Comparative Example 4 | 1.0 | 95 | 0.55 | 135 | 82 | F |
| Comparative Example 5 | 0.9 | 96 | 0.55 | 135 | 82 | F |

### EXAMPLES 7 - 13 & COMPARATIVE EXAMPLES 6 - 10

Cresol novolak resins (a) and (b) obtained in the foregoing paragraphs (1) and (2), respectively, and light-sensitive materials (a) and (b) obtained in the foregoing paragraphs (3) and (4), respectively, and additives (A) to (L) indecated in Table 4 were each dissolved in 15 g of ethylcellosolve acetate in amounts set forth in Table 5, respectively. These solutions were then filtered through a 0.2-µm pore microfilter. The resulting photoresist compositions were each used to obtain 1.2-µm thick resist films in the same manner as in Example 1.

These resist films were each evaluated in the same manner as in Example 1. The results are set forth in Table 5.

**Table 4:**

| Type of Additives | |
|---|---|
| No. | Compound |
| B | Nordihydroguaialetinic acid |
| D | 1,1-(5,5'-Diacetyl-2,3,4,2',3',4'-hexahydroxy) diphenylethane |
| E | p-Bis(2,3,4-trihydroxybenzoylbenzene) |
| F | Bis(2,3,5-trihydroxybenzoyl)methane |
| G | 10,15-Dihydroxy-2,3,7,8,12,13-hexahydroxy-5H-tribenzo[a,d,g] cyclononene |
| H | 2,8,14,20-Tetramethylpentacyclo-[19,3,1,1^{3,7}, 1^{9,13},1^{15,19}]octacosa-1(25),3,5,7(28),9,11, 13(27),15,17,19(26),21,23-dodecaen-4,6,10,12, 16,18,22,24-octol |
| I | 2,3,4-Trihydroxybenzophenone |
| J | 2,3,4,4'-Tetrahydroxybenzophenone |
| K | 2',3,4,3',4',5'-Hexahydroxybenzophenone |
| L | 2,2',4,4'-Tetrahydroxydiphenyl sulfide |

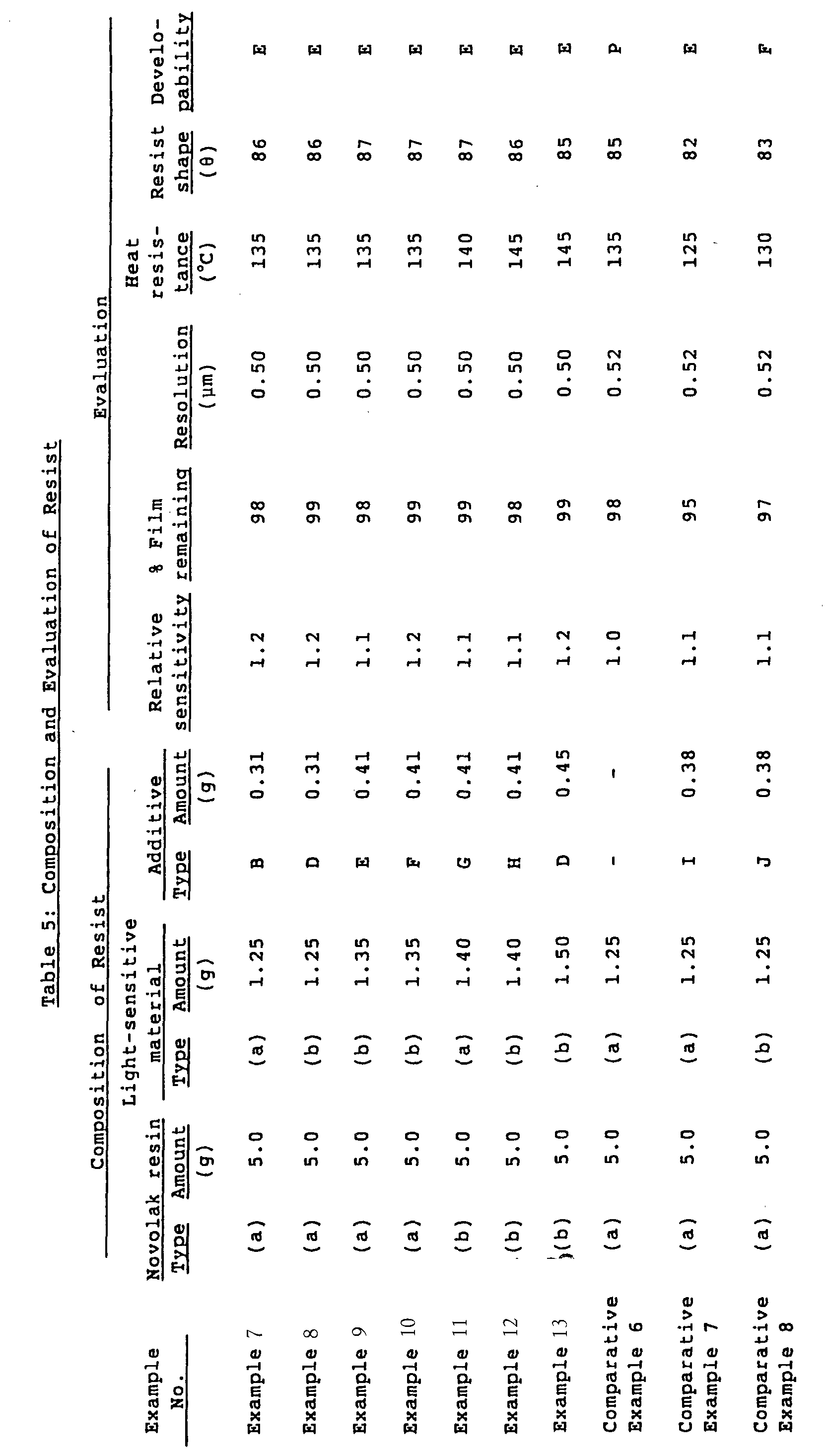

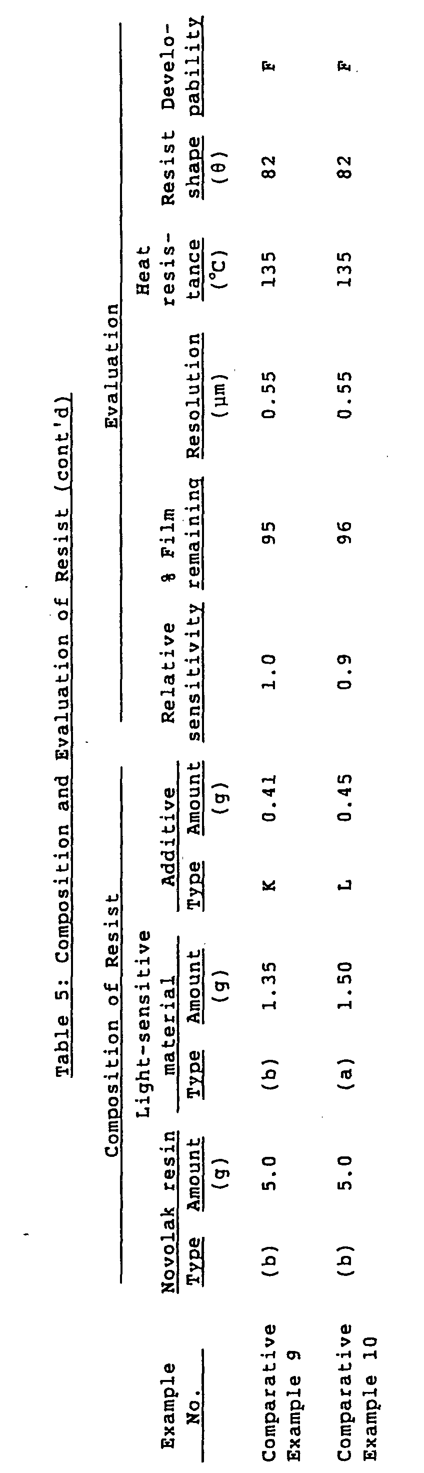

The results show that the resists comprising the additives (A) to (H) of the present invention exhibit excellent sensitivity, percent film remaining, resolution, heat resistance, resist shape and developability.

### EXAMPLES 14-19 & COMPARATIVE EXAMPLES 11 - 15

Cresol novolak resins (a) and (b) obtained in the foregoing parapraphs (1) and (2), respectively, and light- sensitive materials (a) and (b) obtained in the foregoing paragraphs (3) and (4), respectively, and additives (M) to (T) indicated in Table 6 were each dissolved in 15 g of ethylcellosolve acetate in amounts set forth in Table 7, respectively. These solutions were then filtered through a 0.2-µm pore microfilter. The resulting photoresist compositions were each used to obtain 1.2-µm thick resist films in the same manner as in Example 1.

These resist films were each evaluated in the same manner as in Example 1. The results are set forth in Table 7.

**Table 6:**

| Type of Additives | |
|---|---|
| No. | Compound |
| N | 1-(2,3-Dihydroxyphenyl)-1,3,3-trimethyl-4,5-dihydroxyindane |
| O | 2-(3,4,5-Trihydroxyphenyl)-3,5,7-trihydroxybenzopyrane |
| P | 2-(3,4,5-Trihydroxyphenyl)-3-(3,4,5-trihydroxybenzoyloxy)-5, 7-dihydroxybenzopyrane |
| Q | 2,3,4-Trihydroxybenzophenone |
| R | 2,3,4,4'-Tetrahydroxybenzophenone |
| S | 2,3,4,3',4',5'-Hexahydroxybenzophenone |
| T | 2,2',4,4'-Tetrahydroxydiphenyl sulfide |

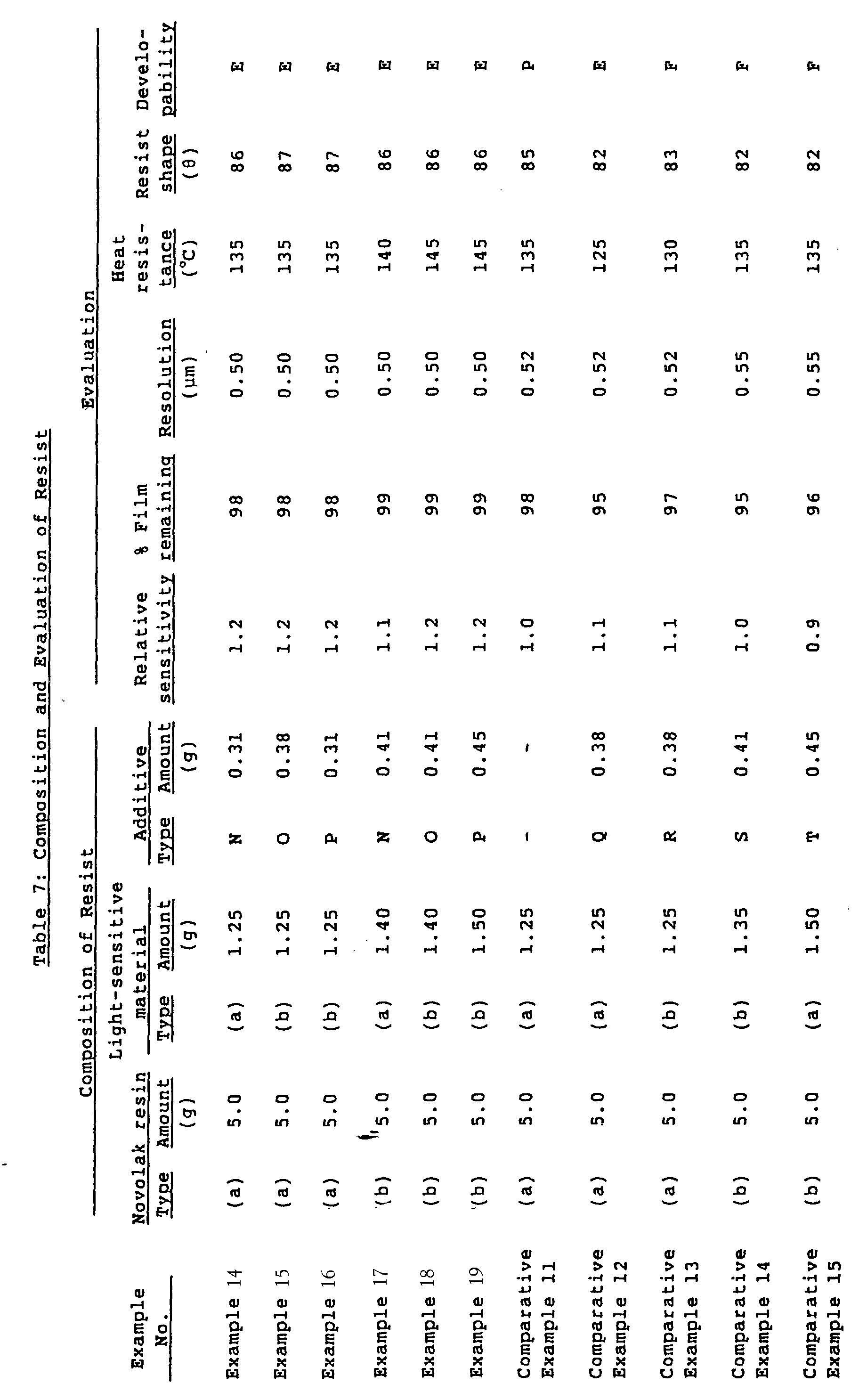

The results show that the resists comprising the additives (M) to (P) of the present invention exhibit excellent sensitivity, percent film remaining, resolution, heat resistance, resist shape and developability.

## Claims

1. A positive type photoresist composition comprising a quinonediazide compound, an alkali-soluble resin, and at least one of the compounds represented by formulae (I) to (VI): 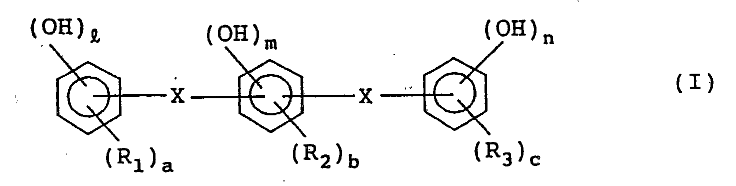 wherein X represents a lower C_{**1-4**} alkylene group; R_{**1**} to R_{**3**} may be the same or different and each represents a hydrogen atom, halogen atom, a C_{**1-4**} alkyl, C_{**1-4**} alkoxy, C_{**2-4**} alkenyl, C_{**2-5**} alkoxycarbonyl, C_{**2-5**} alkyloyloxy, aliphatic C_{**1-5**} acyl, benzoyl or toluoyl group; 1, m and n each represents an integer of from 1 to 3; and a, b and c each represents an integer of from 2 to 4; 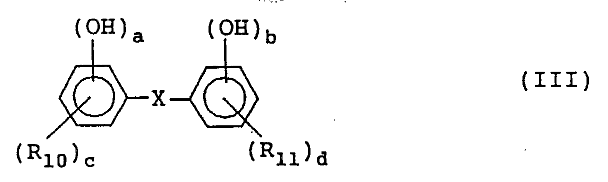 wherein R_{**10**} and R_{**11**} may be the same or different and each represents a hydrogen or halogen atom or a carboxyl, C_{**1-4**} alkyl, phenyl, naphthyl, benzyl, phenethyl, cumyl, benzhydryl, C_{**1-4**} alkoxy, C_{**2-5**} alkoxycarbonyl, C_{**2-5**} alkyloyloxy, aliphatic C_{**1-5**} acyl, benzoyl, toluoyl, phenoxy, naphthoxy, cyano or nitro group each of which may contain substituents; X represents a -CR_{**12**}R_{**13**}- group, 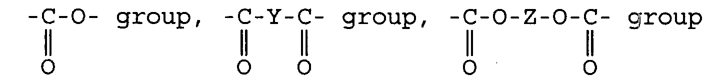 or alkylene group containing 4 or more carbon atoms; Y represents a C_{**1-18**} alkylene group or a benzene residue optionally substituted by C_{**1-4**} alkyl, C_{**1-4**} alkoxy, nitro and halogen; Z represents a C_{**1-4**} alkylene group or an oxyalkylene group derived from ethylene glycol, propylene glycol, polyethylene glycol or polypropylene glycol; R_{**12**} and R_{**13**} each represents (a) an alkyl group containing 5 or more carbon atoms, hydroxyl, phenyl, naphthyl, benzyl, phenethyl, cumyl, benzhydryl, aliphatic C_{**1-5**} acyl, benzoyl or toluoyl group and can form a ring via a carbon bond or ether bond if R_{**10**} and/or R_{**11**} is a hydrogen atom, halogen atom, a C_{**1-4**} alkyl, C_{**1-4**} alkoxy, cyano or nitro group and each group for R_{**12**} and R_{**13**} may contain substituents, or (b) a hydrogen atom, a hydroxyl, C_{**1-10**} alkyl, C_{**1-10**} alkoxy, phenyl, naphthyl, benzyl, phenethyl, cumyl, benzhydryl, aliphatic C_{**1-5**} acyl, benzoyl or toluoyl group and can form a ring via a carbon bond or ether bond if R_{**10**} and R_{**11**} each represents a carboxyl group, phenyl, naphthyl, benzyl, phenethyl, cumyl, benzhydryl, aliphatic C_{**1-5**} acyl, benzoyl, toluoyl, C_{**2-5**} alkoxycarbonyl, C_{**2-5**} alkyloyloxy, phenoxy or naphthoxy group, and each group for R_{**12**} and R_{**13**} may contain substituents; a and b each represents an integer of from 1 to 3, with the proviso that a and b satisfy the relationship 3 ≤ a + b; and c and d each represents an integer of from 1 to 4, with the proviso that a, b, c and d satisfy the relationship a + c = b + d = 5; 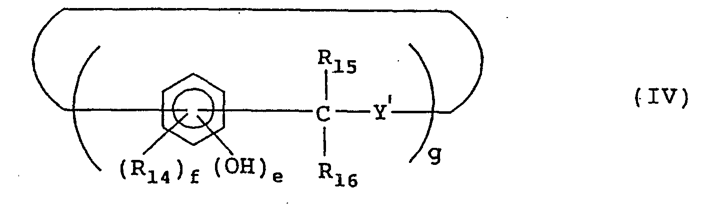 wherein R_{**14**} represents a hydrogen atom, halogen atom, a C_{**1-4**} alkyl, phenyl, naphthyl, benzyl, phenethyl, cumyl, benzhydryl, C_{**1-4**} alkoxy, aliphatic C_{**1-5**} acyl, benzoyl, toluoyl, C_{**2-5**} alkoxycarbonyl, C_{**2-5**} alkyloyloxy, benzoyloxy, toluoyloxy, naphthoyloxy, cyano or nitro group; R_{**15**} and R_{**16**} may be the same or different and each represents a hydrogen atom, a C_{**1-4**} alkyl, phenyl or naphthyl group; Y represents a single bond or -O-CH_{**2**}- group; e and f each represents an integer of from 1 to 3, with the proviso that e and f satisfy the equation e + f = 4; and g represents an integer of from 3 to 8; 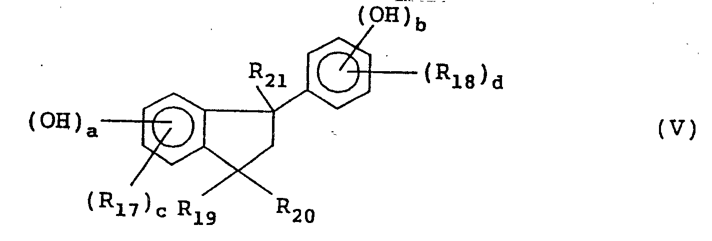 wherein R_{**17**} and R_{**18**} may be the same or different and each represents a hydrogen atom, halogen atom, a C_{**1-4**} alkyl, C_{**1-4**} alkoxy, phenyl, naphthyl, benzyl, phenethyl, cumyl, benzhydryl, amino, C_{**1-4**} monoalkylamino, di- (C_{**1-4**} alkyl)amino, C_{**2-5**} aliphatic acylamino, benzoylamino, toluoylamino, C_{**2-5**} alkylcarbamoyl, phenylcarbamoyl, tolylcarbamoyl, C_{**1-4**} alkylsulfamoyl, carboxyl, cyano, nitro, aliphatic C_{**1-5**} acyl, benzoyl, toluoyl, salicyloyl, naphthoyl, C_{**2-5**} alkyloxycarbonyl, phenoxycarbonyl or aliphatic C_{**2-5**} acyloxy, benzoyloxy, toluoyloxy or naphthoyloxy group each of which may contain substituents; R_{**19**} to R_{**21**} each independently represents a hydrogen atom or a C_{**1-4**} alkyl group which may contain substituents; and a, b, c and d each represents an integer of from 1 to 3, with the proviso that a, b, c and d satisfy the equations a + c = 4 and b + d = 5; 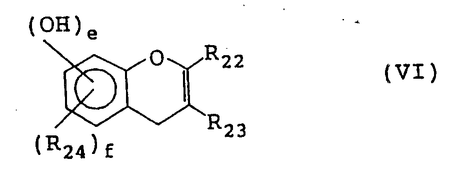 wherein R_{**22**} represents 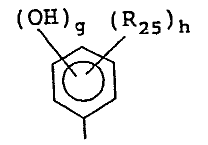 R_{**23**} represents a hydrogen atom, a hydroxyl group, -OR_{**25**} group or 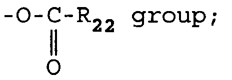 R_{**24**} and R_{**25**} may be the same or different and each represents a hydrogen atom, halogen atom, a C_{**1-4**} alkyl, C_{**1-4**} alkoxy, C_{**2-4**} alkenyl, phenyl, naphthyl, benzyl, phenethyl, cumyl, benzhydryl, aliphatic C_{**1-5**} acyl, benzoyl, toluoyl or C_{**2-5**} alkoxycarbonyl group, each of which may contain substituents; e, f and g may be the same or different and each represents an integer of from 1 to 3; and h represents an integer of from 1 to 4, with the proviso that e, f, g and h satisfy the equations e + f = 4 and g + h = 5;
compounds of formula X-A-H, wherein X is a group of the formula: 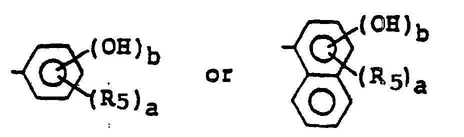 and A is a divalent group of the formula: 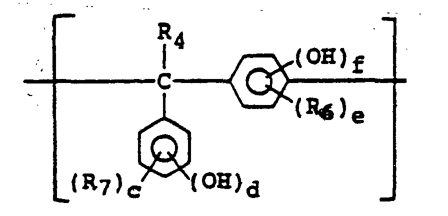 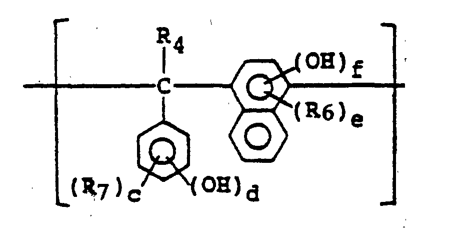 wherein a, b, c, d, e and f are the same or different and a number of 0-3, provided that d+f is not less than 1;
R_{**4**} is hydrogen, C_{**1-18**} alkyl or aryl, R_{**5**}**,** R_{**6**} and R_{**7**} are the same or different and represent C_{**1-18**} alkyl, C_{**1-18**} alkoxy, carboxyl or halogen,
are excluded from the compounds of formula (III).

2. The positive type photoresist composition of claim 1 wherein the proportion of polyhydroxy compound of formula (I) to (VI) to quinonediazide compound is in the range of 150 parts by weight or less per 100 parts by weight of quinonediazide compound.

3. The positive type photoresist composition of claim 2 wherein the proportion of polyhydroxy compound of formula (I) to (VI) to quinonediazide compound is in the range of 5 to 100 parts by weight per 100 parts by weight of quinonediazide compound.

4. The positive type photoresist composition of any one of claims 1 to 3 wherein the alkali-soluble resin is a novolak resin.

5. The positive type photoresist composition of claim 4 wherein the weight average molecular weight of the novolak resin is in the range of 2,000 to 30,000.

## Patentansprüche

1. Positive Photoresist-Zusammensetzung, umfassend eine Chinondiazid-Verbindung, ein Alkali-lösliches Harz und mindestens eine der durch die Formeln (I) bis (VI) dargestellten Verbindungen: 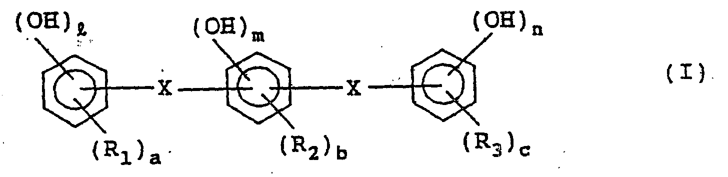 worin X eine C₁₋₄-Niederalkylengruppe ist, R₁ bis R₃ gleich oder verschieden sein können und jeweils ein Wasserstoffatom, Halogenatom, eine C₁₋₄-Alkyl-, C₁₋₄-Alkoxy-, C₂₋₄-Alkenyl-, C₂₋₅-Alkoxycarbonyl-, C₂₋₅-Alkyloyloxy-, aliphatische C₁₋₅-Acyl-, Benzoyl- oder Toluoylgruppe sind, I, m und n jeweils eine ganze Zahl von 1 bis 3 sind und a, b und c jeweils eine ganze Zahl von 2 bis 4 sind, 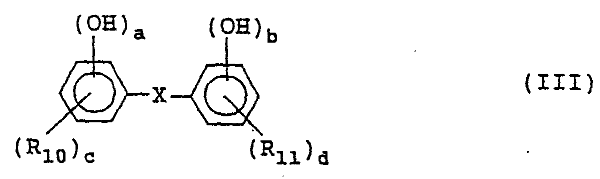 worin R₁₀ und R₁₁ gleich oder verschieden sein können und jeweils ein Wasserstoff- oder Halogenatom oder eine Carboxyl-, C₁₋₄-Alkyl-, Phenyl-, Naphthyl-, Benzyl-, Phenethyl-, Cumyl-, Benzhydryl-, C₁₋₄-Alkoxy-, C₂₋₅-Alkoxycarbonyl-, C₂₋₅-Alkyloyloxy-, aliphatische C₁₋₅-Acyl-, Benzoyl-, Toluoyl-, Phenoxy-, Naphthoxy-, Cyano- oder Nitrogruppe sind, die jeweils Substituenten enthalten können, X eine -CR₁₂R₁₃-Gruppe, 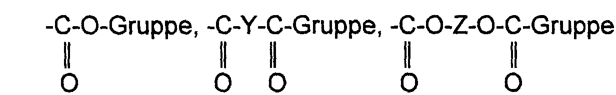 oder Alkylengruppe mit 4 oder mehr Kohlenstoffatomen ist, Y eine C₁₋₁₈-Alkylengruppe oder ein Benzolrest, gegebenenfalls substituiert durch C₁₋₄-Alkyl, C₁₋₄-Alkoxy, Nitro und Halogen, ist, Z eine C₁₋₄-Alkylengruppe oder eine Oxyalkylengruppe, abgeleitet von Ethylenglycol, Propylenglycol, Polyethylenglycol oder Polypropylenglycol, ist, R₁₂ und R₁₃ jeweils (a) eine Alkylgruppe mit 5 oder mehr Kohlenstoffatomen, Hydroxyl-, Phenyl-, Naphthyl-, Benzyl-, Phenethyl-, Cumyl-, Benzhydryl-, aliphatische C₁₋₅-Acyl-, Benzoyl- oder Toluoylgruppe sind und über eine Kohlenstoffbindung oder eine Etherbindung einen Ring bilden können, wenn R₁₀ und/oder R₁₁ ein Wasserstoffatom, Halogenatom, eine C₁₋₄-Alkyl-, C₁₋₄-Alkoxy-, Cyano- oder Nitrogruppe sind, und jede Gruppe für R₁₂ und R₁₃ Substituenten enthalten kann oder (b) ein Wasserstoffatom, eine Hydroxyl-, C₁₋₁₀-Alkyl-, C₁₋₁₀-Alkoxy-, Phenyl-, Naphthyl-, Benzyl-, Phenethyl-, Cumyl-, Benzhydryl-, aliphatische C₁₋₅-Acyl-, Benzoyl- oder Toluoylgruppe sind und über eine Kohlenstoffbindung oder Etherbindung einen Ring bilden können, wenn R₁₀ und R₁₁ jeweils eine Carboxylgruppe, Phenyl-, Naphthyl-, Benzyl-, Phenethyl-, Cumyl-, Benzhydryl-, aliphatische C₁₋₅-Acyl-, Benzoyl-, Toluoyl-, C₂₋₅-Alkoxycarbonyl-, C₂₋₅-Alkyloyloxy-, Phenoxy- oder Naphthoxygruppe sind, und jede Gruppe für R₁₂ und R₁₃ Substituenten enthalten kann, a und b jeweils eine ganze Zahl von 1 bis 3 sind, mit der Maßgabe, daß a und b die Beziehung 3 ≤ a + b erfüllen, und c und d jeweils eine ganze Zahl von 1 bis 4 sind, mit der Maßgabe, daß a, b, c und d die Beziehung a + c = b + d = 5 erfüllen, 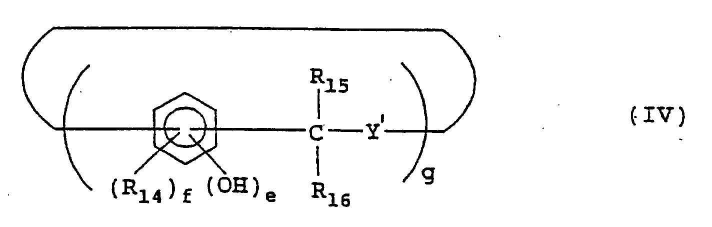 worin R₁₄ ein Wasserstoffatom, Halogenatom, eine C₁₋₄-Alkyl-, Phenyl-, Naphthyl-, Benzyl-, Phenethyl-, Cumyl-, Benzhydryl-, C₁₋₄-Alkoxy-, aliphatische C₁₋₅-Acyl-, Benzoyl-, Toluoyl-, C₂₋₅-Alkoxycarbonyl-, C₂₋₅-Alkyloyloxy-, Benzoyloxy-, Toluoyloxy-, Naphthoyloxy-, Cyano- oder Nitrogruppe ist, R₁₅ und R₁₆ gleich oder verschieden sein können und jeweils ein Wasserstoffatom, eine C₁₋₄-Alkyl-, Phenyl- oder Naphthylgruppe sind, Y eine Einfachbindung oder eine -O-CH₂-Gruppe ist, e und f jeweils eine ganze Zahl von 1 bis 3 sind, mit der Maßgabe, daß e und f die Gleichung e + f = 4 erfüllen, und g eine ganze Zahl von 3 bis 8 ist, 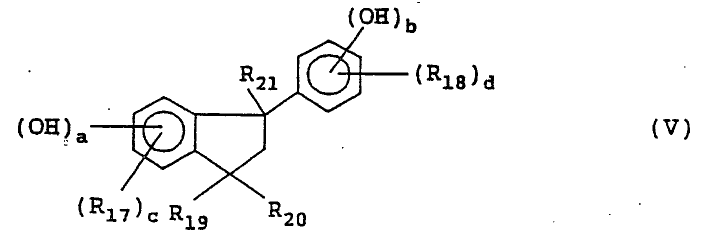 worin R₁₇ und R₁₈ gleich oder verschieden sein können und jeweils ein Wasserstoffatom, ein Halogenatom, eine C₁₋₄-Alkyl-, C₁₋₄-Alkoxy-, Phenyl-, Naphthyl-, Benzyl-, Phenethyl-, Cumyl-, Benzhydryl-, Amino-, C₁₋₄-Monoalkylamino-, Di-(C₁₋₄-alkyl)amino-, aliphatische C₂₋₅-Acylamino-, Benzoylamino-, Toluoylamino-, C₂₋₅-Alkylcarbamoyl-, Phenylcarbamoyl-, Tolylcarbamoyl-, C₁₋₄-Alkylsulfamoyl-, Carboxyl-, Cyano-, Nitro-, aliphatische C₁₋₅-Acyl-, Benzoyl-, Toluoyl-, Salicyloyl-, Naphthoyl-, C₂₋₅-Alkyloxycarbonyl-, Phenoxycarbonyl- oder aliphatische C₂₋₅-Acyloxy-, Benzoyloxy-, Toluoyloxy- oder Naphthoyloxygruppe sind, die jeweils Substituenten enthalten können, R₁₉ bis R₂₁ jeweils unabhängig ein Wasserstoffatom oder eine C₁₋₄-Alkylgruppe, die Substituenten enthalten kann, sind, und a, b, c und d jeweils eine ganze Zahl von 1 bis 3 sind, mit der Maßgabe, daß a, b, c und d die Gleichungen a + c = 4 und b + d = 5 erfüllen, 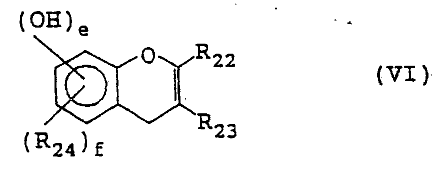 worin R₂₂ 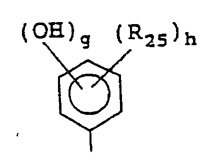 ist, R₂₃ ein Wasserstoffatom, eine Hydroxylgruppe, -OR₂₅-Gruppe oder 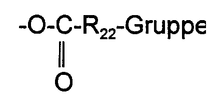 ist, R₂₄ und R₂₅ gleich oder verschieden sein können und jeweils ein Wasserstoffatom, ein Halogenatom, eine C₁₋₄-Alkyl-, C₁₋₄-Alkoxy-, C₂₋₄-Alkenyl-, Phenyl-, Naphthyl-, Benzyl-, Phenethyl-, Cumyl-, Benzhydryl-, aliphatische C₁₋₅-Acyl-, Benzoyl-, Toluoyl- oder C₂₋₅-Alkoxycarbonylgruppe sind, die jeweils Substituenten enthalten können, e, f und g gleich oder verschieden sein können und jeweils eine ganze Zahl von 1 bis 3 darstellen und h eine ganze Zahl von 1 bis 4 ist, mit der Maßgabe, daß e, f, g und h die Gleichungen e + f = 4 und g + h = 5 erfüllen,
Verbindungen der Formel X-A-H, worin X eine Gruppe der Formel 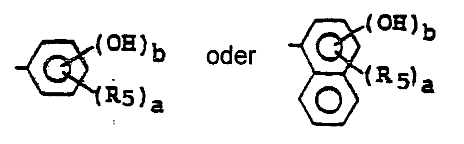 ist und A eine zweiwertige Gruppe der Formel: 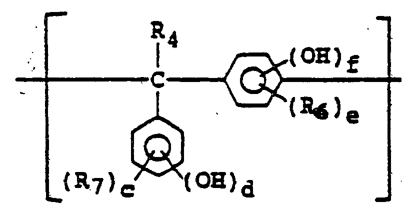 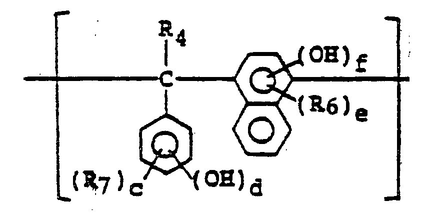 ist, worin a, b, c, d, e und f gleich oder verschieden sind und eine Zahl von 0 bis 3 sind, mit der Maßgabe, daß d + f nicht kleiner als 1 ist, R₄ Wasserstoff, C₁₋₁₈-Alkyl oder Aryl ist, R₅, R₆ und R₇ gleich oder verschieden sind und C₁₋₁₈-Alkyl, C₁₋₁₈-Alkoxy, Carboxyl oder Halogen darstellen,
von den Verbindungen der Formel (III) ausgenommen sind.

2. Positive Photoresist-Zusammensetzung nach Anspruch 1, worin das Verhältnis von Polyhydroxy-Verbindung von Formel (I) bis (VI) zur Chinondiazid-Verbindung im Bereich von 150 Gew.-Teilen oder weniger pro 100 Gew.-Teile Chinondiazid-Verbindung liegt.

3. Positive Photoresist-Zusammensetzung nach Anspruch 2, worin das Verhältnis von Polyhydroxy-Verbindung von Formel (I) bis (VI) zur Chinondiazid-Verbindung im Bereich von 5 bis 100 Gew.-Teilen pro 100 Gew.-Teile Chinondiazid-Verbindung liegt.

4. Positive Photoresist-Zusammensetzung nach irgendeinem der Ansprüche 1 bis 3, worin das Alkali-lösliche Harz ein Novolak-Harz ist.

5. Positive Photoresist-Zusammensetzung nach Anspruch 4, worin das Gewichtsmittel des Molekulargewichts des Novolak-Harzes im Bereich von 2.000 bis 30.000 liegt.

## Revendications

1. Composition de photorésist positif, comprenant un composé de type diazooxyde, une résine soluble en milieu alcalin, et au moins un composé représenté par l'une des formules (I) à (VI) : 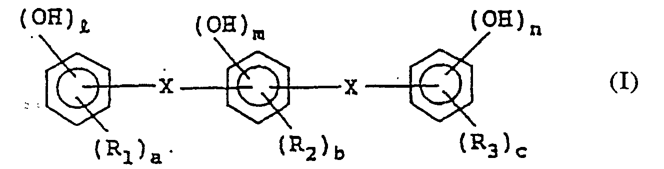 dans laquelle
X représente un groupe alkylène inférieur en C₁₋₄,
les symboles R₁ à R₃ peuvent avoir des significations identiques ou différentes et représentent chacun un atome d'hydrogène ou d'halogène ou un groupe alkyle en C₁₋₄, alcoxy en C₁₋₄, alcényle en C₂₋₄, alcoxycarbonyle en C₂₋₅, alcanoyloxy en C₂₋₅, acyle aliphatique en C₁₋₅, benzoyle ou toluyle,
l, m et n représentent chacun un nombre entier valant de 1 à 3, et a, b et c représentent chacun un nombre entier valant de 2 à 4 ; 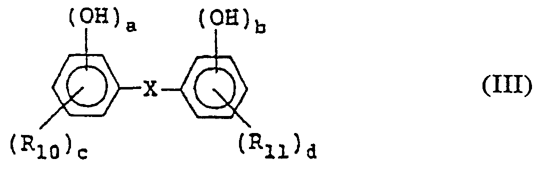 dans laquelle
R₁₀ et R₁₁ peuvent avoir des significations identiques ou différentes et représentent chacun un atome d'hydrogène ou d'halogène ou un groupe carboxy, alkyle en C₁₋₄, phényle, naphtyle, benzyle, phénéthyle, cumyle, benzhydryle, alcoxy en C₁₋₄, alcoxycarbonyle en C₂₋₅, alcanoyloxy en C₂₋₅, acyle aliphatique en C₁₋₅, benzoyle, toluyle, phénoxy, naphtoxy, cyano ou nitro, chacun de ces groupes pouvant porter des substituants,
X représente un groupe de formule -CR₁₂R₁₃-, -C(=O)-O-, -C(=O)-Y-C(=O)- ou -C(=O)-O-Z-O-C(=O)-, ou un groupe alkylène comportant au moins 4 atomes de carbone,
Y représente un groupe alkylène en C₁₋₁₈ ou un groupe phénylène portant éventuellement un substituant alkyle en C₁₋₄, alcoxy en C₁₋₄, nitro ou halogéno,
Z représente un groupe alkylène en C₁₋₄ ou un groupe oxyalkylène dérivé d'éthylèneglycol, de propylèneglycol, de polyéthylèneglycol ou de polypropylèneglycol,
R₁₂ et R₁₃ représentent chacun
a) un groupe alkyle comportant au moins 5 atomes de carbone, hydroxy, phényle, naphtyle, benzyle, phénéthyle, cumyle, benzhydryle, acyle aliphatique en C₁₋₅, benzoyle ou toluyle, lequel groupe peut constituer un cycle, par l'intermédiaire d'une liaison carbone-carbone ou d'une liaison de type éther, dans le cas où R₁₀ et/ou R₁₁ représentent un atome d'hydrogène ou d'halogène ou un groupe alkyle en C₁₋₄, alcoxy en C₁₋₄, cyano ou nitro, chacun des groupes représentés par R₁₂ et R₁₃ pouvant porter des substituants, ou bien
b) un atome d'hydrogène ou un groupe hydroxy, alkyle en C₁₋₁₀, alcoxy en C₁₋₁₀, phényle, naphtyle, benzyle, phénéthyle, cumyle, benzhydryle, acyle aliphatique en C₁₋₅, benzoyle ou toluyle, lequel groupe peut constituer un cycle, par l'intermédiaire d'une liaison carbone-carbone ou d'une liaison de type éther, dans le cas où R₁₀ et/ou R₁₁ représentent chacun un groupe carboxy, phényle, naphtyle, benzyle, phénéthyle, cumyle, benzhydryle, acyle aliphatique en C₁₋₅, benzoyle, toluyle, alcoxycarbonyle en C₂₋₅, alcanoyloxy en C₂₋₅, phénoxy ou naphtoxy, chacun des groupes représentés par R₁₂ et R₁₃ pouvant porter des substituants, et
a et b représentent chacun un nombre entier valant de 1 à 3, sous réserve que a et b satisfassent la relation 3 ≤ (a + b), et c et d représentent chacun un nombre entier valant de 1 à 4, sous réserve que a, b, c et d satisfassent les égalités (a + c) = (b + d) = 5 ; 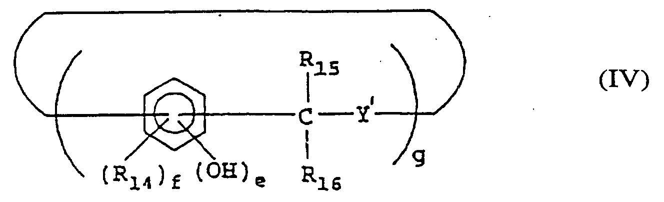 dans laquelle
R₁₄ représente un atome d'hydrogène ou d'halogène ou un groupe alkyle en C₁₋₄, phényle, naphtyle, benzyle, phénéthyle, cumyle, benzhydryle, alcoxy en C₁₋₄, acyle aliphatique en C₁₋₅, benzoyle, toluyle, alcoxycarbonyle en C₂₋₅, alcanoyloxy en C₂₋₅, benzoyloxy, toluyloxy, naphtoyloxy, cyano ou nitro,
R₁₅ et R₁₆ peuvent avoir des significations identiques ou différentes et représentent chacun un atome d'hydrogène ou un groupe alkyle en C₁₋₄, phényle ou naphtyle,
Y représente une liaison simple ou un groupe -O-CH₂-, et
e et f représentent chacun un nombre entier valant de 1 à 3, sous réserve que e et f satisfassent l'égalité (e + f) = 4, et g représente un nombre entier valant de 3 à 8 ; 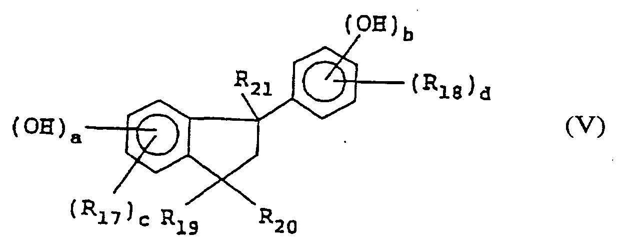 dans laquelle
R₁₇ et R₁₈ peuvent avoir des significations identiques ou différentes et représentent chacun un atome d'hydrogène ou d'halogène ou un groupe alkyle en C₁₋₄, alcoxy en C₁₋₄, phényle, naphtyle, benzyle, phénéthyle, cumyle, benzhydryle, amino, mono(alkyle en C₁₋₄)amino, di(alkyle en C₁₋₄)amino, (acyle aliphatique en C₂₋₅)amino, benzoylamino, toluylamino, alkylcarbamyle en C₂₋₅, phénylcarbamyle, tolylcarbamyle, alkylsulfamyle en C₁₋₄, carboxy, cyano, nitro, acyle aliphatique en C₁₋₅, benzoyle, toluyle, salicyle, naphtoyle, alcoxycarbonyle en C₂₋₅, phénoxycarbonyle, acyloxy aliphatique en C₂₋₅, benzoyloxy, toluyloxy ou naphtoyloxy, chacun de ces groupes pouvant porter des substituants,
les symboles R₁₉ à R₂₁ représentent chacun, indépendamment, un atome d'hydrogène ou un groupe alkyle en C₁₋₄ qui peut porter des substituants, et
a, b, c et d représentent chacun un nombre entier valant de 1 à 3, sous réserve que a, b, c et d satisfassent les égalités (a + c) = 4 et (b + d) = 5 ; 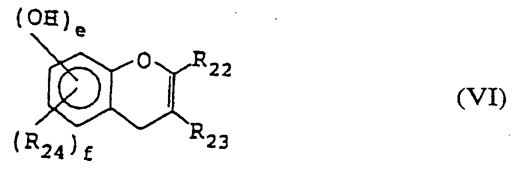 dans laquelle
R₂₂ représente un groupe de formule 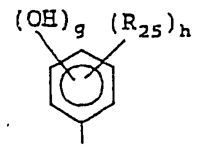
R₂₃ représente un atome d'hydrogène, un groupe hydroxy ou un groupe de formule -OR₂₅ ou -O-C(=O)-R₂₂,
R₂₄ et R₂₅ peuvent avoir des significations identiques ou différentes et représentent chacun un atome d'hydrogène ou d'halogène ou un groupe alkyle en C₁₋₄, alcoxy en C₁₋₄, alcényle en C₂₋₄, phényle, naphtyle, benzyle, phénéthyle, cumyle, benzhydryle, acyle aliphatique en C₁₋₅, benzoyle, toluyle ou alcoxycarbonyle en C₂₋₅, chacun de ces groupes pouvant porter des substituants,
e, f et g représentent chacun un nombre entier valant de 1 à 3 et peuvent avoir des valeurs identiques ou différentes, et h représente un nombre entier valant de 1 à 4, sous réserve que e, f, g et h satisfassent les égalités (e + f) = 4 et (g + h) = 5 ;
étant exclus des composés de formule (III) les composés de formule X-A-H dans laquelle X représente un groupe de formule 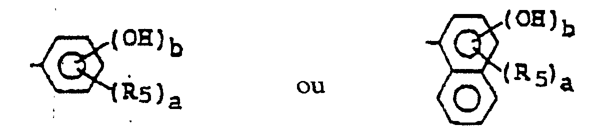 et A représente un groupe divalent de formule 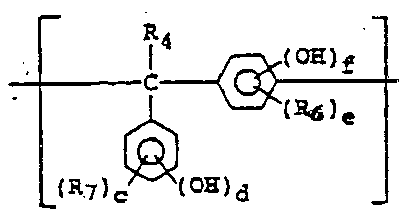 ou 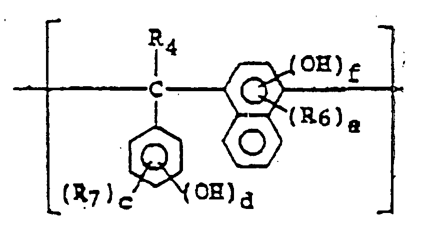 où a, b, c, d, e et f représentent des nombres identiques ou différents et valant de 0 à 3, sous réserve que (d + f) vaille au moins 1, R₄ représente un atome d'hydrogène ou un groupe alkyle en C₁₋₁₈ ou aryle, et R₅, R₆ et R₇ peuvent avoir des significations identiques ou différentes et représentent chacun un groupe alkyle en C₁₋₁₈, alcoxy en C₁₋₁₈ ou carboxy ou un atome d'halogène.

2. Composition de photorésist positif, conforme à la revendication 1, dans laquelle la proportion de composé polyhydroxylé représenté par l'une des formules (I) à (VI) au composé de type diazooxyde est d'au plus 150 parties en poids pour 100 parties en poids de diazooxyde.

3. Composition de photorésist positif, conforme à la revendication 1, dans laquelle la proportion de composé polyhydroxylé représenté par l'une des formules (I) à (VI) au composé de type diazooxyde se trouve dans l'intervalle allant de 5 à 100 parties en poids pour 100 parties en poids de diazooxyde.

4. Composition de photorésist positif, conforme à l'une des revendications 1 à 3, dans laquelle la résine soluble en milieu alcalin est une résine novolaque.

5. Composition de photorésist positif, conforme à la revendication 4, dans laquelle la masse molaire moyenne en poids de la résine novolaque vaut de 2 000 à 30 000.
